# EUROPEAN PATENT APPLICATION

(11) **EP 2 052 715 A1**
(43) Date of publication of application: **29.04.2009**
(21) Application number: 07792255.7
(22) Date of filing: 09.08.2007
(51) Int. Cl.: A61K 8/25, A61K 8/02, A61K 8/06, A61K 8/894, B28C 1/04

(54) **SOL COMPOSITION CONTAINING ORGANOMODIFIED CLAY MINERAL HAVING FLAKY PARTICLE STRUCTURE, OIL-BASE GEL COMPOSITIONS AND W/O EMULSION COMPOSITIONS CONTAINING THE SAME**

(30) Priority: 09.08.2006 JP 2006216656; 09.08.2006 JP 2006216657
(71) Applicant: SHISEIDO COMPANY LIMITED, Chuo-ku, Tokyo 104-8010 (JP)
(72) Inventor: UEDA, Hideto, Yokohama-shi Kanagawa 224-8558 (JP); NASU, Akio, Yokohama-shi Kanagawa 224-8558 (JP)
(74) Representative: Henkel, Feiler & Hänzel
(86) International application number: PCT/JP2007/065603
(87) International publication number: WO 2008/018540

(57) **Abstract**

The present invention is to provide a sol composition, a oily gel composition, and a W/O type emulsion comprising an organically-modified clay mineral, having a platy particle structure with an average thickness of 0.1 µm or less and an average major axis of 0.5 to 50 µm in oil. It showed that the thicknening and gelling ability becomes significantly high, under the presence of a nonionic surfactant, by elaminating the aggregate of organically-modified clay mineral into platy particles in oil. In addition, because the stabilizing effect of a W/O emulsion is excellent, it is possible to provide a W/O emulsion with high emulsion stability at low viscosity by lowering the usage of the organically-modified clay mineral with a platy particle structure and the nonionic surfactant. Furthermore, it is possible to lower the viscosity of the composition, while maintaining the emulsion stability, by using an oil having COOH/OH groups. Thus, an emulsion with excellent stability at low viscosity and with an excellent feeling in use, such as spreadability and freshness, can be obtained.

## Description

### RELATED APPLICATIONS

This application claims the priority of Japanese Patent Application No. 2006-216656 and Japanese Patent Application No. 2006-216657 filed on August 9, 2006, which are incorporated herein by reference.

### TECHNICAL FIELD

The present invention relates to a sol composition, an oily gel composition, and a W/O type emulsion composition containing an organically-modified clay mineral having a platy particle structure, and in particular, relates to the thickening/gelling ability of the organically-modified clay mineral to an oil component, stabilizing ability in a W/O emulsion, and the improvement of the feeling in use of the compositions.

### BACKGROUND ART

In the past, W/O emulsions have been used in various products. In particular, silicone oil is an important oil in the cosmetic field because it has a smooth property and it is excellent in volatility and water-repellent properties, and it is certainly desired to be blended in W/O type cosmetics. However, it is difficult to form stable W/O emulsions with silicone oil.

From the standpoint of the feeling in use and the handling, the oil thickening and gelation have been conducted not only in cosmetics and quasi-drugs but also in various fields such as paint and resin. For example, polymer thickeners and wax have been used. Among oils, silicone oil is especially important in the cosmetic field.

Thus far, for the gelation of silicone oil and for the stabilization of W/O emulsions containing silicone oil, a solidification of silicone oil with waxes, and a combined use with silica or hydrophilized silica has been adopted.

However, the solidification method with waxes had a problem in the feeling in use in that the spreadability was heavy and it was sticky. The application range was also limited because of poor fluidity. In addition, a method of concomitant use of silica had a problem in that the long-term stability was poor.

In order to improve the usability and long-term stability, a method to treat an organically-modified clay mineral with a polyether-modified silicone compound and blend it to gel compositions and W/O emulsions has been developed (for example, refer to patent literatures 1 and 2).

However, the gel formation ability of the gel composition containing the organically-modified clay mineral treated with the above-described polyether-modified silicone compound was not long-lasting. In addition, the function as an emulsifier when it was blended into W/O emulsions was not satisfactory.

Furthermore, if we try to secure the emulsion stability by the use of the conventionally used organically-modified clay mineral, the viscoelasticity, among gel characteristics, generated by the necessary amount of the component tends to be relatively high, thus it was very difficult to obtain an emulsion with low viscosity and excellent stability.
Patent literature 1: Japanese Unexamined Patent Publication No. S61-114721
Patent literature 2: Japanese Unexamined Patent Publication No. S61-212321

### DISCLOSURE OF THE INVENTION

### PROBLEM TO BE SOLVED BY THE INVENTION

The present invention was made in view of the above-described circumstances, and an object is to provide an organically-modified clay mineral thickening/gelling agent that can achieve excellent thickening/gelling ability of oil and to provide oily gel compositions containing the same.
Another object is to provide an organically-modified clay mineral emulsifier that can achieve an excellent long-term stabilizing effect, such as the suppression of time-dependent aggregation/coalescence of emulsion particles, when used in W/O emulsions and to provide W/O emulsions containing the same.

Another object is to provide an organically-modified clay mineral emulsifier that can achieve a W/O emulsion stabilizing effect at low viscosity and can generate W/O emulsions excellent in the feeling in use and to provide W/O emulsions containing the same.

### MEANS TO SOLVE THE PROBLEM

The present invention was made in view of the above-described circumstances, and an object is to provide an organically-modified clay mineral thickening/gelling agent that can achieve excellent thickening/gelling ability of oil and to provide oily gel compositions containing the same.
Another object is to provide an organically-modified clay mineral emulsifier that can achieve an excellent long-term stabilizing effect, such as the suppression of time-dependent aggregation/coalescence of emulsion particles, when used in W/O emulsions and to provide W/O emulsions containing the same.
Another object is to provide an organically-modified clay mineral emulsifier that can achieve a W/O emulsion stabilizing effect at low viscosity and can generate W/O emulsions excellent in the feeling in use and to provide W/O emulsions containing the same.

The gel formation ability of the gel composition containing the organically-modified clay mineral treated with the above-described polyether-modified silicone compound is not long-lasting, and the performance as an emulsifier is not satisfactory when it is blended in a W/O emulsion. These points are considered as follows.
That is, the shape of clay mineral is essentially that of layered (laminated) platy particles with a thickness of a few nm. Commercial organically-modified clay minerals are aggregated during the production process; thus the layered clay minerals are further aggregated, and the average thickness is normally 2 µm or higher. Accordingly, the aggregate of clay mineral cannot be made microscopic or plate-like only by the treatment with polyether-modified silicone compounds. Thus, organically-modified clay mineral cannot be sufficiently dispersed; as a result, a stable gel network structure may not be formed in oil. This is considered to be the cause of the poor performance as a gelling agent or emulsifier.

The present inventors have conducted a study based on such an idea. As a result, it has been clarified that the thickening/gelling ability becomes significantly high, under the presence of a nonionic surfactant, by delaminating the aggregate of organically-modified clay mineral in oil with a mechanical shearing force and/or impact force and controlling the shape into microscopic platy particles. In addition, the stabilizing effect of W/O emulsions becomes very high. Thus, it becomes possible, by reducing the usage of the organically-modified clay mineral platy particles and the nonionic surfactant, to provide a W/O emulsion excellent in the stability at relatively low viscosity.

The present inventors have further conducted a study; as a result, the present inventors have found the following, thus leading to completion of the present invention. If an oil having a COOH group and/or OH group in the molecule is applied to the oily gel composition that contains organically-modified clay mineral platy particles and a nonionic surfactant, the emulsion stabilizing effect can be maintained even though the viscosity of the oily gel composition and the viscosity of the W/O emulsion containing the same decrease. Thus, the feeling in use such as spreadability and freshness can be improved compared with the use of the aggregate of the conventional organically-modified clay mineral.

That is, the sol composition of the present invention is characterized by comprising an organically-modified clay mineral having a platy particle structure with an average thickness of 0.1 µm or less and an average major axis of 0.5 to 50 µm in oil. In the present invention, respective platy particles are essentially not aggregated.
In the sol composition of the present invention, it is preferable that the above-described organically-modified clay mineral is an organically-modified hectorite.
In the sol composition of the present invention, it is also preferable that the oil contains silicone oil.
In the sol composition of the present invention, it is also preferable that the production is carried out by delaminating, in oil, the aggregate of organically-modified clay mineral having a layer structure with an average thickness of 2 µm or more with a mechanical shearing force and/or impact force.

The oily gel composition of the present invention is characterized by comprising any of the above-described sol compositions and a nonionic surfactant.
The oily gel composition of the present invention is also characterized by comprising the below-described components (i) to (iii).
(i) Organically-modified clay mineral, having a platy particle structure, with an average thickness of 0.1 µm or less and an average major axis of 0.5 to 50 µm
(ii) Nonionic surfactant
(iii) Oil
In the oily gel composition of the present invention, it is preferable that the above-described organically-modified clay mineral having a platy particle structure is an organically-modified hectorite.
In the oily gel composition of the present invention, it is also preferable that the oil contains silicone oil.

In the oily gel composition of the present invention, it is also preferable that the production is carried out by delaminating, in oil, the aggregate of organically-modified clay mineral having a layer structure with an average thickness of 2 µm or more with a mechanical shearing force and/or impact force in the presence of a nonionic surfactant.
In the oily gel composition of the present invention, it is also preferable that the production is carried out by delaminating, in oil, the aggregate of organically-modified clay mineral having a layer structure with an average thickness of 2 µm or more, in the absence of a nonionic surfactant, with a mechanical shearing force and/or impact force, and the subsequent addition of a nonionic surfactant.

In the oily gel composition of the present invention, it is also preferable that the nonionic surfactant is a polyether-modified silicone compound.
In any of the above-described oily gel composition, it is also preferable that an oil having a COOH group and/or OH group in the molecule is additionally contained.

In the oily gel composition of the present invention, it is also preferable that the production is carried out by delaminating, in oil, the aggregate of organically-modified clay mineral having a layer structure with an average thickness of 2 µm or more, in the absence of an oil having a COOH group and/or OH group in the molecule, with a mechanical shearing force and/or impact force, and the subsequent addition of an oil having a COOH group and/or OH group in the molecule.

In the oily gel composition of the present invention, it is also preferable that the production is carried out by delaminating, in the oil containing an oil having a COOH group and/or OH group in the molecule, the aggregate of organically-modified clay mineral having a layer structure with an average thickness of 2 µm or more with a mechanical shearing force and/or impact force.

In the oily gel composition of the present invention, it is also preferable that the blending quantity of the above-described organically-modified clay mineral having a platy particle structure is 0.25 to 30 mass % of the oily gel composition, and it is more preferable that the blending quantity of the above-described organically-modified clay mineral having a platy particle structure is 0.25 to 10 mass % of the oily gel composition.

In the oily gel composition of the present invention, it is also preferable that the blending quantity of the above-described nonionic surfactant is 0.1 to 10 mass % of the oily gel composition.
In the oily gel composition of the present invention, it is also preferable that the content of the oil having a COOH group and/or OH group in the molecule is 0.1 to 5 mass % of the oily gel composition.
The W/O type emulsion composition or the cosmetics of the present invention are characterized in that any of the above-described oily gel compositions is used.

### EFFECT OF THE INVENTION

The organically-modified clay mineral with a platy particle structure of the present invention can achieve an excellent thickening and gelling effect of oil in combination use with a nonionic surfactant. In addition, because the stabilizing effect of a W/O emulsion is excellent, it is possible to provide a W/O emulsion with high emulsion stability at low viscosity by lowering the usage of the organically-modified clay mineral with a platy particle structure and the nonionic surfactant. Furthermore, it is possible to lower the viscosity of the composition, while maintaining the emulsion stability, by using an oil having COOH/OH groups. Thus, an emulsion with excellent stability at low viscosity and with an excellent feeling in use, such as spreadability and freshness, can be obtained.

### BRIEF DESCRIPTION OF THE DRAWINGS

Fig. 1 shows an SEM micrograph of a commercial organically-modified clay mineral.
Fig.2 shows an SEM micrograph of an organically-modified clay mineral having a platy particle structure.
Fig.3 schematically shows the formation of a gel network, in silicone oil, for the aggregate of organically-modified clay mineral and for the organically-modified clay mineral having a platy particle structure.
Fig.4 shows the pictures of dispersion states before and after the addition of a polyether-modified silicone compound for the case in which an oily gel composition was prepared by the high-dispersion treatment of a commercial organically-modified clay mineral (gel composition 1-3).
Fig.5 shows the pictures of dispersion states before and after the addition of a polyether-modified silicone compound for the case in which an oily gel composition was prepared by the normal dispersion treatment of a commercial organically-modified clay mineral (gel composition 1-4).
Fig. 6 shows a relationship between the storage modulus (G') and the shear stress for the oily gel composition in which an organically-modified clay mineral having a platy particle structure of the present invention was used.
Fig. 7 shows a relationship between the storage modulus (G') and the shear stress for the oily gel composition in which a commercial organically-modified clay mineral treated by normal dispersion was used.
Fig. 8 shows a relationship between the storage modulus (G') and the shear stress for the oily gel composition in which an organically-modified clay mineral having a platy particle structure of the present invention was used.
Fig. 9 shows a relationship between the storage modulus (G') and the shear stress for the oily gel composition in which a commercial organically-modified clay mineral treated by normal dispersion was used.
Fig. 10 shows the swelling properties of oily gel compositions 2-3 and 2-4 by changing the amount of added polyether-modified silicone compound.
Fig. 11 shows an SEM micrograph of oil dispersion 3-4 obtained by the normal dispersion treatment of a commercial organically-modified clay mineral.
Fig. 12 shows an SEM micrograph of oil dispersion 3-3 obtained by the high-dispersion treatment of a commercial organically-modified clay mineral.
Fig. 13 shows the viscosity variation, of oil dispersions, due to the concentration of oil having COOH/OH groups.

### BEST MODE FOR CARRYING OUT THE INVENTION

### (1) Sol composition containing an organically-modified clay mineral

The sol composition of the present invention is an oil dispersion, in which organically-modified clay mineral particles are dispersed in oil, and contains organically-modified clay mineral particles having a platy particle structure with an average thickness of 0.1 µm or less and an average major axis of 0.5 to 50 µm. Respective platy particles are essentially not aggregated.
The above-described organically-modified clay mineral with an average thickness of 2 µm or more can be obtained by laminating, in oil, the aggregate of organically-modified clay mineral with a layer structure with a mechanical shearing force and/or impact force. Specifically, it is obtained as a fluid sol composition, by the following treatment method, normally at low viscosity.

### (Treatment method)

To a mixture of a commercial organically-modified clay mineral (normally an aggregate with an average thickness of 2 µm or more and with a layer structure) and oil, the same volume of glass beads (or zirconia beads etc.) with a diameter of about 1 mm are added. The delamination was carried out with a paint shaker (Asada Iron Works Co., Ltd.), bead mill (Dispermat, VMA-Getzmann GmbH Verfahrenstechnik), etc. and by exerting a mechanical shearing force and/or impact force.

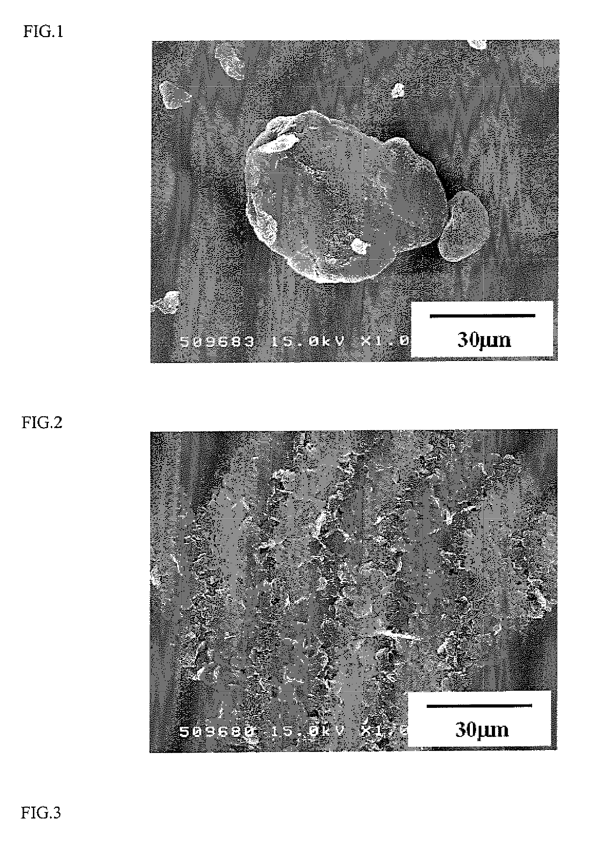

An SEM micrograph of the commercial organically-modified hectorite (Bentone 38 VCG, manufactured by Elementis Specialties, Inc. (UK)), which was used as raw material (Fig. 1), and an SEM micrograph of the organically-modified hectorite with the platy particle structure (Fig. 2) obtained by treating with the above-described treatment method are shown. (After the dispersion in the blending ratio of decamethylcyclopentasiloxane: organically-modified hectorite = 95:5, photographs were taken with an SEM S-4500 (manufactured by Hitachi High-Technologies Corporation))
The above-described delamination treatment can be performed in the presence of the below-described nonionic surfactant and/or the oil having COOH/OH groups.

The organically-modified clay mineral of the present invention has a platy shape with an average thickness of 0.1 µm or less because of the above-described treatment. In the conventional clay mineral, platy particles are layered. In a commercial organically-modified clay mineral that is prepared by the organic modification of the layered platy particles, the clay mineral particles with the layer structure are further aggregated and the appearance is shown in Fig. 1.

In the present invention, a sol composition, in which organically-modified clay mineral particles having a platy structure with an average thickness of 0.1 µm or less are independently present as isolated particles, can be obtained by the delamination of this aggregate. The once obtained dispersion can maintain a good dispersion state, without reaggregation, in the oil such as silicone oil. If the aggregate is not sufficiently delaminated and the thickness remains large, the below-described thickening and gelling ability and the stabilizing ability of W/O emulsion cannot be satisfactorily achieved.
The average major axis of the organically-modified clay mineral contained in the sol composition of the present invention is preferably 0.5 to 50 µm. Individual particle shapes are not limited in particular and need not be angular so far as a sheet structure is taken within the range of the above-described thickness and major axis length.

The average thickness and the average major axis of the organically-modified clay mineral particles contained in the sol composition of the present invention can be determined, for example, by the following measurement methods.

### (Measurement method)

### • Average thickness

The sol composition is sufficiently diluted with silicone oil, and a dried sample of the diluted solution is measured with a scanning electron microscope (S-4500, manufactured by Hitachi High-Technologies Corporation).

### • Average major axis

The sol composition is sufficiently diluted with silicone oil, and the measurement is conducted with a particle size distribution analyzer (Microtrac VSR, manufactured by Nikkiso Co., Ltd.).
The shape of the above-described organically-modified clay mineral is determined from the SEM micrograph.

When the organically-modified clay mineral having a platy particle structure of the present invention is prepared, an organically-modified clay mineral used as the raw material is not limited in particular and any organically-modified clay mineral can be used so far as it is commonly used for cosmetics. Specifically, they are obtained by ion-exchanging the interlayer cation of a water-swelling clay mineral with a cationic surfactant such as an alkyl quaternary ammonium salt, and the examples include those exchanged with benzyl dimethyl stearyl ammonium ion and those exchanged with dimethyl distearyl ammonium ion. Specific examples of water-swelling clay minerals include montmorillonite, smectite, and hectorite. In the present invention, a preferable organically-modified clay mineral is the above-described organically-modified hectorite. Examples of commercial organically-modified clay minerals include Bentone 27 and Bentone 38 (manufactured by Elementis Specialties, Inc. (UK)).

In the sol composition of the present invention, the blending quantity of the above-described organically-modified clay mineral having a platy structure is not limited in particular. However, the blending quantity is preferably 0.25 to 35 mass %. If the blending quantity is too much, the delamination treatment may become difficult.

The oil used in the above-described treatment method is not limited in particular. One or more oils normally used in cosmetics, pharmaceuticals, etc. can be used. It is preferable that the entirety is liquid oil at ordinary temperature (20 °C). In the present invention, in particular, silicone oil is preferably used.

There are no particular limitations on the silicone oil so far as it is normally used in cosmetics. Specific examples include linear polysiloxanes such as dimethylpolysiloxane and methylphenylpolysiloxane; cyclic polysiloxanes such as octamethylcyclotetrasiloxane, decamethylcyclopentasiloxane, and dodecamethylcyclohexasiloxane; and silicone resins with a three-dimensional network and silicone rubber various polysiloxanes (amino-modifiled polysoloxane, alkyl-modified polysiloxane, and fluorine-modified lolysioxane, etc.) so far as there are no special issues.
In the present invention, one or more silicone oils may be selected for use.

The organically-modified clay mineral having a platy structure of the present invention can be especially well dispersed in the oil in which silicone oil is the main component, and it is possible to effectively carry out thickening/gelling in combination with nonionic surfactants such as polyether-modified silicone compounds. In addition, it is possible to achieve an excellent emulsion stabilizing effect of W/O emulsion compositions in which silicone oil is the main component.

Examples of oils other than silicone oils include hydrocarbon oils, ester oils, plant-derived oils, animal-derived oils, higher alcohols, and higher fatty acids.

Examples of hydrocarbon oils include liquid paraffin, paraffin, squalane, squalene, ozokerite, pristane, ceresin, petrolatum, and microcrystalline wax.

Examples of ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, glyceryl trioctanoate, glyceryl triisopalmitate, trimethylolpropane triisostearate, cetyl 2-ethylhexanoate, pentaerythritol tetra-2-ethylhexanoate, glyceryl tri-2-ethylhexanoate, 2-ethylhexyl palmitate, glyceryl trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of plant-derived oils include avocado oil, camellia oil, macadamia nut oil, corn oil, olive oil, rapeseed oil, sesame oil, castor oil, peanut oil, almond oil, soybean oil, tea seed oil, jojoba oil, and germ oil.
Examples of animal-derived oils include turtle oil, egg oil, and mink oil.
Examples of higher alcohols include oleyl alcohol, isostearyl alcohol, octyldodecanol, decyltetradecanol, jojoba alcohol, cetyl alcohol, and myristyl alcohol, and the examples of higher fatty acids include oleic acid, isostearic acid, linoleic acid, linolenic acid, eicosapentaenoic acid, docosahexaenoic acid, palmitic acid, and stearic acid.

### (2) Gel composition containing an organically-modified clay mineral

When a nonionic surfactant is used in combination with the above-described sol composition, the viscosity significantly increases compared with the sol composition and the fluidity decreases. In the present invention, this kind of composition is called a gel composition. Thus, the gel composition of the present invention can be said to be more thickened oil dispersion than the above-described sol composition (a dispersion obtained by removing the nonionic surfactant from the gel composition).

The addition of the nonionic surfactant can be before the delamination treatment of the organically-modified clay mineral in oil, during the delamination treatment, or after the delamination treatment.
For example, the oily gel composition of the present invention can be obtained by the addition of a nonionic surfactant to the above-described sol composition.
Alternatively, the oily gel composition of the present invention can be obtained by the delamination of the aggregate of organically-modified clay mineral (normally, an average thickness of 2 µm or more) in oil with a mechanical shearing force and/or impact force in the presence of a nonionic surfactant. Even in the oily gel composition by such a method, the above-described organically-modified clay mineral having a platy particle structure can be formed.
The viscosity of the gel composition and the W/O emulsion containing the same tend to be lower in the case of the oily gel composition that is prepared, as in the former method, by the addition of the nonionic surfactant after obtaining the sol composition by the high-dispersion treatment, in oil, of the organically-modified clay mineral than the case of the oily gel composition prepared in one step, as in the latter method; in addition, the viscosity lowering effect by the below-described oil having COOH/OH groups tend to be higher.
The average thickness and the average major axis of the organically-modified clay mineral particles contained in the oily gel composition of the present invention can be determined, for example, by the following measurement methods.

### (Measurement method)

### • Average thickness

The oily gel composition is sufficiently diluted with silicone oil, and a dried sample of the diluted solution is measured with a scanning electron microscope (S-4500, manufactured by Hitachi High-Technologies Corporation).

### • Average major axis

The oily gel composition is sufficiently diluted with silicone oil, and the measurement is conducted with a particle size distribution analyzer (Microtrac VSR, manufactured by Nikkiso Co., Ltd.).
For these measurements, the nonionic surfactant in the oily gel composition may be washed with silicone oil.
In the present invention, for the above-described measurement of the average thickness and the average major axis, the sol composition, in which the organically-modified clay mineral is dispersed in silicone oil and diluted, can be used as a measurement sample.
The shape of the above-described organically-modified clay mineral is determined from the SEM micrograph.

In the oily gel composition of the present invention, the nonionic surfactant adsorbs on the platy particle surface of the above-described organically-modified clay mineral having a platy structure and contributes to the formation of a stable gel network. Because the nonionic surfactant is adsorbed on the surface of microscopic platy particles, the adsorption rate is improved. Thus, the blending effect can be achieved with a smaller amount of the nonionic surfactant than in the gel composition where the conventional organically-modified clay mineral aggregate is used.
In addition, when a W/O type emulsion composition is prepared with the use of the oily gel composition of the present invention, finely dispersed particles of organically-modified clay mineral, the surface of which is improved with a nonionic surfactant, form the membrane wall of emulsion particles, and the emulsion composition with excellent stability can be formed.

Nonionic surfactants used in the present invention include those normally used for pharmaceuticals, cosmetics, and the like.
Specific examples include ethylene oxide addition type surfactants including ether-type activators such as POE (2-50) oleyl ether, POE (2-40) stearyl ether, POE (2-50) lauryl ether, POE (1-50) alkylphenyl ether, POE (5-30) behenyl ether, POE (5-25) 2-decylpentadecyl ether, POE (3-20) 2-decyltetradecyl ether, and POE (5-25) 2-octyldodecyl ether; ester-type activators such as POE (4-100) hydrogenated castor oil, POE (3-60) castor oil, POE (2-150) fatty acid monoester, POE (2-150) fatty acid diester, and POE (5-20) sorbitan fatty acid ester; and ether/ester-type activators such as POE (2-60) glyceryl monoisostearate, POE (3-60) glyceryl triisostearate, and POE (5-60) hydrogenated castor oil triisostearate; and polyhydric alcohol fatty acid ester surfactants including polyglycerin fatty acid esters such as decaglyceryl tetraoleate, diglyceryl diisostearate, decaglyceryl tetraisostearate, decaglyceryl tetrastearate, decaglyceryl heptaoleate, decaglyceryl decaoleate, and decaglyceryl decaisostearate; and glycerin fatty acid esters such as glyceryl monostearate, glyceryl monoisostearate, and glyceryl monooleate. One or more nonionic surfactants can be selected for use.

As a preferable nonionic surfactant of the present invention, polyether-modified silicone compounds can be listed. As polyether-modified silicone compounds, the below-described polyoxyalkylene-modified organopolysiloxane represented by general formulas (I) to (VII) can be listed.

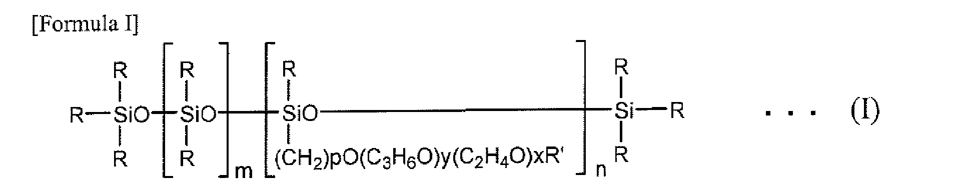

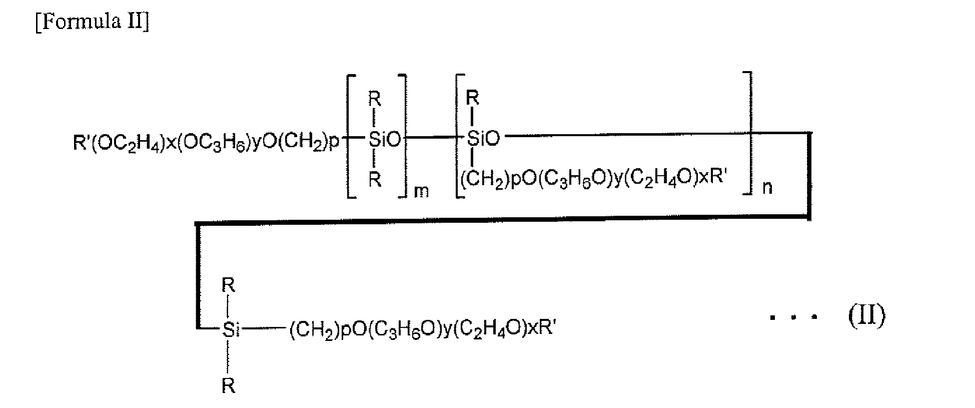

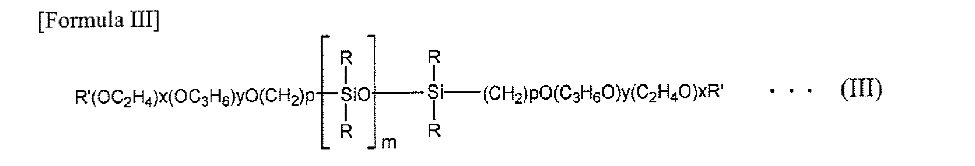

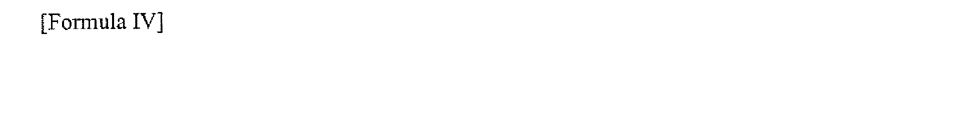

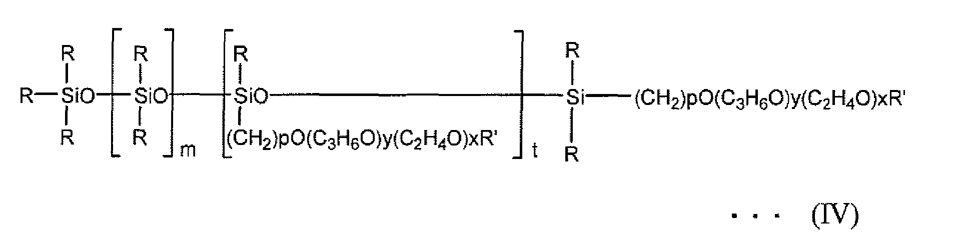

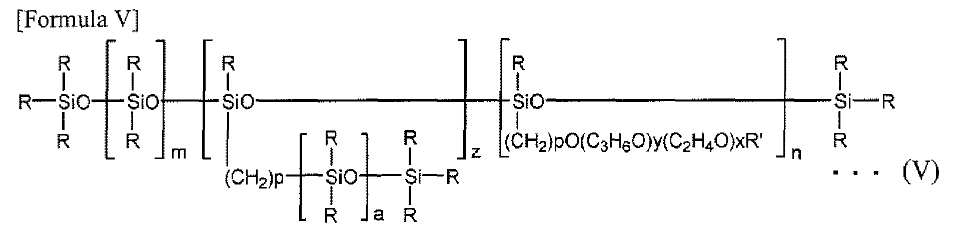

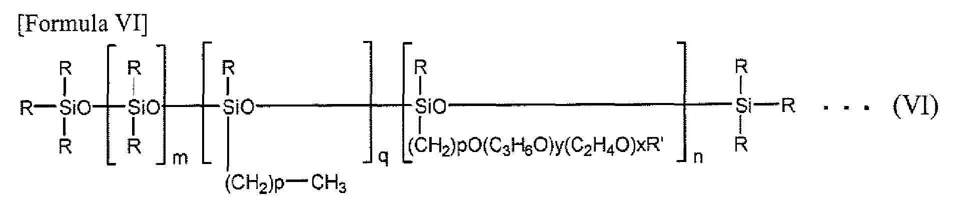

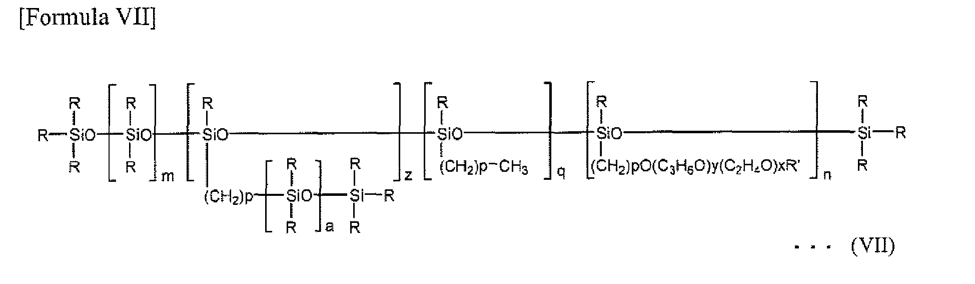

(In general formulas (I) to (VII), R is an alkyl group or phenyl group having 1 to 3 carbon atoms, R' is an hydrogen atom or an alkyl group having 1 to 12 carbon atoms, p is an integer from 1 to 50, m and a are integers from1 to 100, n, q, x, and z are integers from 1 to 50, and t and y are integers from 0 to 50.)

The use of polyether-modified silicone compounds represented by the above-described general formulas (I) to (VII) is especially preferable when the oil contained in the oily gel composition of the present invention is silicone oil. In the present invention, it is preferable that one or more polyether-modified silicone compounds, which are represented above, are arbitrarily selected for use.
It is possible to use commercial products as the compounds represented by the above-described general formulas (I) to (VII), and polyoxyethylene/methylpolysiloxane copolymers etc. can be listed.
In the oily gel composition of the present invention, the preferable blending quantity of silicone oil is 20 to 90 mass % of the total amount of the gel composition.

In the oily gel composition of the present invention, the preferable blending quantity of the nonionic surfactant is 0.1 to 10 mass % of the total amount of the oily gel composition.
In the oily gel composition, the mass ratio of the organically-modified clay mineral having a platy structure and the nonionic surfactant is preferable to be "organically-modified clay mineral having a platy structure" : "nonionic surfactant" = 1:1 to 10:1.
If the amount of the nonionic surfactant is too small, the effect may not be satisfactorily achieved. On the other hand, if the nonionic surfactant is used too much, the marked improvement in the effect cannot be expected, and rather a negative effect may be caused to the feeling in use.

In the oily gel composition of the present invention, the blending quantity of the organically-modified clay mineral having a platy particle structure is preferably 0.25 to 30 mass % of the total amount of the oily gel composition, more preferably 0.25 to 10 mass %, and the most preferably 0.5 to 5 mass %. If the blending quantity is less than 0.25 mass %, a blending effect cannot be observed. If the blending quantity exceeds 30 mass %, a hard gel tends to be formed in combination use with a nonionic surfactant, and the improved effect due to an increase cannot be observed.

In the sol composition and oily gel composition of the present invention, it is possible to include any other oil component so far as there are no special issues, and silicone oil and other oils can be added as necessary.
For example, the preferable oily gel composition of the present invention contains an organically-modified clay mineral with a platy particle structure, a polyether-modified silicone compound, and silicone oil as the main components. It is possible to add oil other than silicone oil.
Examples of other oils include hydrocarbon oil, ester oil, plant-derived oil, animal-derived oil, higher alcohols, and higher fatty acids. It is preferable that these oils dissolve in silicone oil, and the entirety becomes a homogeneous liquid at ordinary temperature (20 °C).
In the oily gel composition of the present invention, the preferable blending quantity of oil is 70 mass % or higher of the total amount of the oily gel composition.

Among these oils, if especially an oil having a COOH group and/or OH group in the molecule (sometimes called "oil having COOH/OH groups" in the present invention) is used, the viscosity can be lowered without losing the stability of gel compositions and that of W/O emulsions containing the same. Accordingly, in the W/O emulsion containing the above-described organically-modified clay mineral platy particles and nonionic surfactant in the oil phase, low viscosity and good spreadability can be achieved for the W/O emulsion by using an oil having COOH/OH groups in the oil phase even though the emulsion stability comparable to the case of no oil use is maintained. In addition, the feelings in use such as spreadability, freshness, and smoothness of this W/O emulsion are very good compared with the case in which the conventional organically-modified clay mineral aggregate is used.
This reason is not clear, however, it is considered that the oil having COOH/OH groups weaken the bonding strength without destroying the gel network structure of the organically-modified clay mineral platy particles.

The content of an oil having COOH/OH groups is preferably 0.1 to 5 mass % of the oily gel composition, and more preferably 0.5 to 3 mass %. If the amount of the oil having COOH/OH groups is too small, the effect may not be satisfactory. On the other hand, if an excess is blended, a marked effect may not be achieved, and the emulsion stability and the feeling in use may be adversely affected.

As an oil having COOH/OH groups, any oil can be used so far as the oil is normally used for pharmaceuticals, cosmetics, etc., and an oil that is soluble in silicone oil and liquid at ordinary temperature is preferable. Examples include liquid higher fatty acids such as isostearic acid; liquid higher alcohols such as isostearyl alcohol; higher fatty acids; and polyhydric alcohol fatty acid esters such as sorbitan sesquiisostearate.

When an oil having COOH/OH groups is blended in an oily gel composition, the addition can be carried out at any stage, namely, before, during, or after the delamination treatment of organically-modified clay mineral; however, it is preferable, from the standpoint of effectiveness, to add after the delamination treatment.
When an oil having COOH/OH groups is blended in a W/O emulsion composition, the oil can be added after emulsification; however, the addition to the oil phase is preferably carried out before emulsification.

### (3) W/O emulsion composition

In the following, the W/O emulsion composition of the present invention is explained.
Water and water-soluble components are blended in the water phase that constitutes the W/O type emulsion composition.
On the other hand, in the oil phase, the above-described oily gel composition is used as the main component.

The W/O type emulsion composition of the present invention is produced by the ordinary production method of W/O type emulsion compositions. For example, the W/O type emulsion composition of the present invention can be obtained by the addition of the water phase to the oil phase containing the above-described oily gel composition and the emulsification thereof.
In the total amount of the W/O type emulsion composition, it is preferable that the ratio of the above-described water phase:oil phase is 80:20 to 5:95.

In the oily gel composition or W/O type emulsion composition of the present invention, other components commonly used for cosmetics can be suitably blended.
Examples include drugs, various surfactants, inorganic powder, organic powder, pigments, colorants, moisturizers, thickeners, metal sequestering agents, various water-soluble polymer, pH adjusters, antioxidants, and preservatives.
The oily gel composition or W/O type emulsion composition of the present invention can be used as a base for the conventional oil gel cosmetics and W/O emulsion cosmetics. Examples include skin care cosmetics such as milky lotion, cream, cleanser, pack, and massage cosmetics; makeup cosmetics such as foundation, lipstick, cheek color, eyeliner, and eyeshadow; sunscreen cosmetics; and hair cosmetics such as hair treatment and hair dressing; however, they are not limited to these examples.

### [Examples]

Hereinafter, the present invention will be described in more detail with reference to specific examples. However, the present invention is not limited by these examples. Unless otherwise noted, the blending quantity is expressed in mass %.

### I. Sol compositions containing an organically-modified clay mineral

Initially, the preparation method of sol compositions of an organically-modified clay mineral used in the following examples is shown.

### Sol compositions 1-1 to 1-4

Commercial organically-modified hectorite (Bentone 38 VCG, manufactured by Elementis Specialties, Inc. (UK)) (15 mass %) and decamethylcyclopentasiloxane (85 mass %) were mixed. To this mixture, glass beads with a diameter of 1 mm were added in the same volume as the mixture, and the delamination treatment was carried out by mechanically applying a high shearing force and/or impact force with a bead mill. Sol compositions 1-1 to 1-3 were obtained by varying the treatment time (5 minutes, 10 minutes, and 15 minutes), respectively.
As a comparative example, sol composition 1-4 was obtained by treating with a Disper disperser, instead of a bead mill, for 10 minutes without adding glass beads.

The particle shapes of organically-modified clay minerals contained in the obtained sol composition are shown in Table 1. The measurement of the average thickness and the average major axis was carried out as follows.

### (Measurement method)

### • Average thickness

The sol composition was sufficiently diluted with decamethyleyclopentasiloxane, and a dried sample of the diluted solution was measured with a scanning electron microscope (S-4500, manufactured by Hitachi High-Technologies Corporation).

### • Average major axis

The sol composition was sufficiently diluted with decamethylcyclopentasiloxane, and the measurement was conducted with a particle size distribution analyzer (Microtrac VSR, manufactured by Nikkiso Co., Ltd.).

**[Table 1]**

| | Shape | Average thickness (µ m) | Average major axis (µ m) |
|---|---|---|---|
| Sol composition 1-1 | Thickness | 0.05 | 9.5 |
| Sol composition 1-2 | Thickness | 0.05 | 5.7 |
| Sol compositions 1-3 | Thickness | 0.04 | 5.2 |
| Sol composition 1-4 | Glanular | Average particle size 19.9 | |

In sol composition 1-4 obtained by the normal dispersion treatment with a Disper disperser, the organically-modified clay mineral remained as relatively large granules (aggregate) as seen from the SEM micrograph in Fig. 1. On the other hand, in sol compositions 1-1 to 1-3 obtained by high-dispersion treatment with a bead mill, the organically-modified clay mineral was delaminated to microscopic platy particles as seen in Fig. 2.

### II. Oily gel compositions containing an organically-modified clay mineral

In addition, oily gel compositions 1-1 to 1-4 were prepared using sol compositions 1-1 to 1-4, which were prepared as above, of an organically-modified clay mineral. Production examples are shown below.

### Oily gel compositions 1-1 to 1-4

Respective sol compositions 1-1 to 1-4 of the above-described organically-modified clay mineral (33.33 weight portion), polyoxyethylene/methylpolysiloxane copolymer (general formula (I) type, polyoxyethylene content: 20%, viscosity: 400 to 800 cs, HLB: 4.5, R' = H, p = 3, y = 0) (2.5 weight portion), and decamethylcyclopentasiloxane (3.5 to 4.5 cs) (64.17 weight portion) were dispersion-treated with a Disper disperser for 10 minutes, and oily gel compositions 1-1 to 1-4 were obtained (the concentration of the organically-modified clay mineral in each oily gel composition was 5 mass %).

The swelling property was investigated for the above-described oily gel compositions 1-1 to 1-4. The results are shown in Table 2. The evaluation method and evaluation criteria for swelling property were as follows.

### (Swelling property of oily gel compositions)

Samples were placed in transparent glass bottles, and each oily gel composition was visually evaluated based on the following criteria.
○: No change such as liquid phase separation was observed.
△: 1/5 or less of the total gel composition is separated.
× : 1/5 or more of the total gel composition is separated.

**[Table 2]**

| | Sol composition | Swelling property |
|---|---|---|
| Oily gel composition 1-1 | 1-1 | △ |
| Oily gel composition 1-2 | 1-2 | ○ |
| Oily gel composition 1-3 | 1-3 | ○ |
| Oily gel composition 1-4 | 1-4 | × |

As is clear from the results in Table 2, the swelling property of oily gel composition 1-4 obtained by using oily sol composition 1-4, which was obtained by the treatment with a Disper disperser, and a nonionic surfactant was poor.
On the other hand, in oily gel compositions 1-1 to 1-3 obtained by using sol compositions 1-1 to 1-3 obtained by the treatment with a bead mill and a nonionic surfactant, the high swelling property was obtained compared with oily gel composition 1-4. In addition, the swelling property of the oily gel composition improved with an increase in the treatment time of the sol composition to 10 minutes and to 15 minutes. This is considered because the microscopic delamination of the organically-modified clay mineral progresses with treatment time as shown in Table 1. In particular, the organically-modified clay mineral contained in oily gel composition 1-3 had a platy particle structure, with good dispersibility, with an average thickness of 0.1 µm or less and an average major axis of about 5 µm.

The particle shape of the organically-modified clay mineral contained in oily gel composition 1-4 was granular, and it was different from the shape of the above-described organically-modified clay mineral having a platy particle structure.
In the above-described organically-modified clay mineral with a platy particle structure, the platy particle surface (flat side) tends to be hydrophobic, and the end surface tends to be hydrophilic. Therefore, it is arranged in silicone oil so that the exposure of the hydrophilic end surface is reduced. If schematically expressed, it is considered that a gel network shown in Fig. 3 is formed and the high swelling property can be achieved.
On the other hand, in the granular organically-modified clay mineral particles, the formation of a gel network due to the contribution of hydrophilic and hydrophobic portions is difficult; as a result, the swelling property is considered to be poor.

In the organically-modified clay mineral contained in sol compositions 1-1 to 1-3, the average major axis and the dispersibility of platy particles are different depending upon treatment time. It was clarified from the above-described results that an excellent swelling property can be obtained by a delamination treatment time of about 15 minutes or longer.

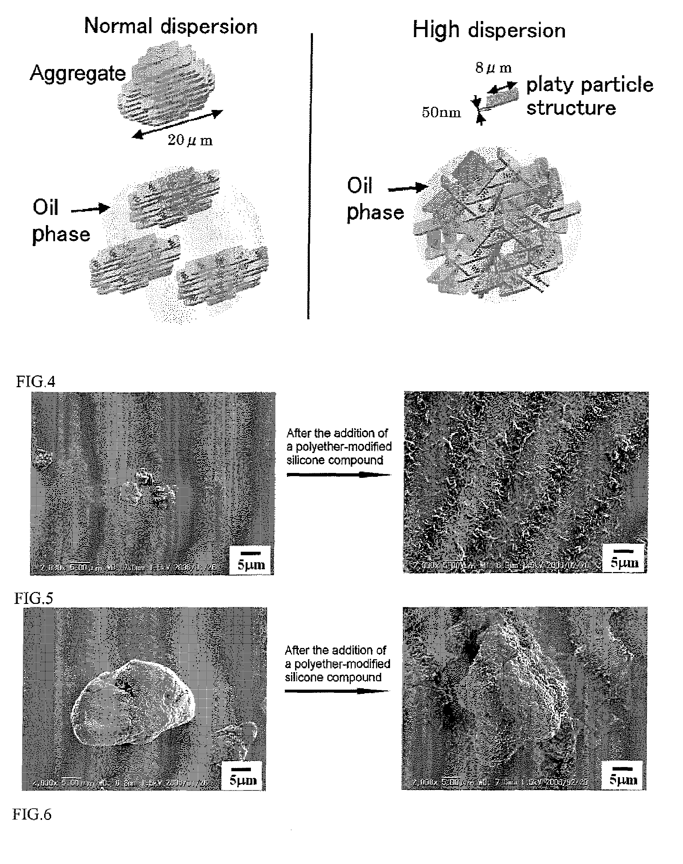

In the production process of the above-described oily gel compositions 1-3 and 1-4, the dispersion states of the organically-modified clay mineral before and after the addition of a polyether-modified silicone, which is a nonionic surfactant, were recorded on SEM micrographs (refer to Fig. 4 and Fig. 5).
From the SEM micrographs, when an oily gel composition was prepared using the organically-modified clay mineral having a platy particle structure, which is contained in the sol composition of the present invention (Fig. 4), the particles of clay mineral are clearly dispersed in a microscopic state both before and after the addition of a polyether-modified silicone compound.
On the other hand, when the granular organically-modified clay mineral (aggregate) by the normal dispersion treatment was used (Fig. 5), the aggregate of the organically-modified clay mineral became somewhat microscopic by the addition of a polyether-modified silicone compound; however, large aggregates were still observed and the dispersion state was not satisfactory.

In addition, the stability of a gel structure of the above-described oily gel composition was evaluated from the measurement of dynamic viscoelasticity. The measurement method was as follows.

### (Dynamic viscoelasticity measurement)

### Measurement apparatus: Rheometer AR1000-N (TA Instruments)

Measurement method: The measurement of dynamic viscoelasticity was conducted by using a corn plate. Under the condition of a measurement frequency of 1 Hz, the gel storage modulus, G' [Pa], with respect to the shear stress (0.01-50 [Pa]) was measured.

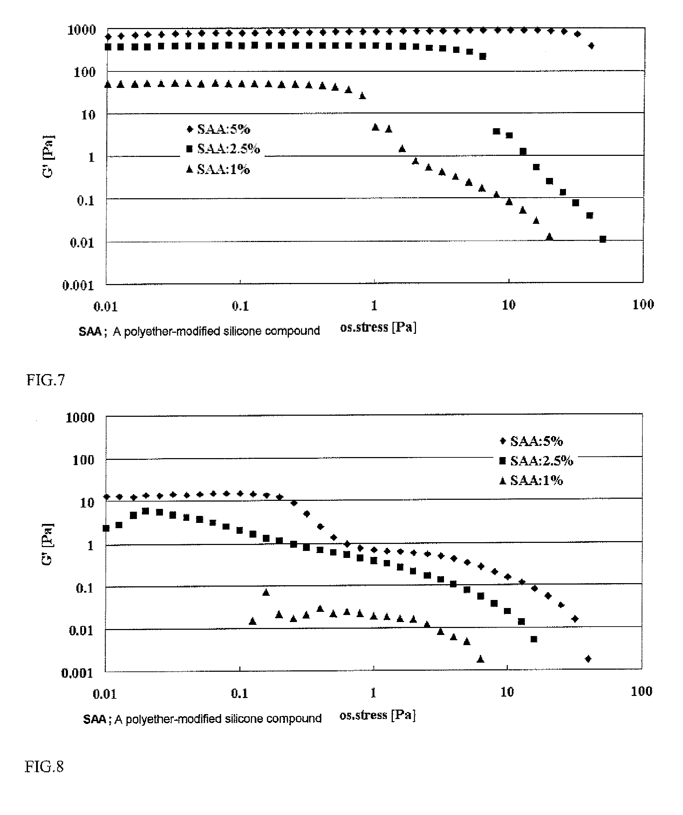

The relationship between the storage modulus (G') and the shear stress for oily gel composition 1-3 (organically-modified clay mineral: platy particles) of the present invention is shown in Fig. 6, and the relationship for oily gel composition 1-4 (organically-modified clay mineral: aggregate) is shown in Fig. 7. In respective figures, the cases in which the concentration of polyether-modified silicone compound contained in respective oily gel compositions was varied are also shown (the increase and decrease were adjusted with decamethylcyclopentasiloxane).

As is for oily gel composition 1-3 of the present invention, when the organically-modified clay mineral particles are platy, it was confirmed that the gel elasticity was high within the fixed shear stress range, a constant state was maintained, and the stable gel structure was taken (Fig. 6).
On the other hand, as is for oil gel 1-4, when the granular organically-modified clay mineral (aggregate) by the normal dispersion treatment of a commercial organically-modified clay mineral is used, the gel elasticity was small, and a stable region with a constant gel elasticity state was scarce compared with the above-described case in Fig. 6.

From the above results, the organically-modified clay mineral having a platy particle structure, which is contained in the sol composition of the present invention, achieves an excellent effect in the formation of a stable gel structure in oil under the presence of a nonionic surfactant. Thus, it is very clear that the thickening and gelling effect is improved compared with the organically-modified clay mineral with the conventional aggregate structure.

The above trend is also similar in the oily gel composition that is obtained, in one step without going through a sol composition, by the delamination of an organically-modified clay mineral, in oil, under the presence of a nonionic surfactant (refer to the below-described oily gel compositions 2-1 to 2-4).

### Oily gel compositions 2-1 to 2-4

To each commercial organically-modified hectorite (Bentone 38 VCG, manufactured by Elementis Specialties, Inc. (UK)) (5 weight portions), polyoxyethylene/methylpolysiloxane copolymer (general formula (I) type, polyoxyethylene content: about 20%, viscosity: 400 to 800 cs, HLB: 4.5, R' = H, p = 3, y = 0) (2.5 weight portion), and decamethylcyclopentasiloxane (92.5 weight portion) was added the same volume of glass beads (or zirconia beads etc.), and oily gel compositions 2-1 to 2-3 were obtained by stirring and homogenizing by a mechanical shearing force and/or impact force with a bead mill for 5 minutes, 10 minutes, and 15 minutes, respectively.
In addition, as a comparative example, oily gel composition 2-4 was obtained by conducting normal dispersion treatment for 10 minutes, without beads, with a Disper disperser instead of a bead mill.

The particle shapes of the organically-modified clay mineral contained in oily gel compositions 2-1 to 2-4 were observed with SEM. The organically-modified clay mineral in oily gel composition 2-4 was present as aggregates similar to those of the above-described oily gel composition 1-4, and the organically-modified clay mineral in oily gel compositions 2-1 to 2-3 had a microscopic platy-particle structure similar to that of the above-described oily gel compositions 1-1 to 1-3.

The swelling property were investigated for the above-described oily gel compositions 2-1 to 2-4. The results are shown in Table 3.

**[Table 3]**

| | Swelling property |
|---|---|
| Oily gel composition 2-1 | △ |
| Oily gel composition 2-2 | ○ |
| Oily gel composition 2-3 | ○ |
| Oily gel composition 2-4 | × |

As is clear from the results in Table 3, oily gel composition 2-4 obtained by the treatment with a Disper disperser had a poor swelling property.
On the other hand, the swelling property of oily gel compositions 2-1 to 2-3 that was treated with a bead mill improved with an increase in treatment time to 10 minutes and to 15 minutes. In particular, the organically-modified clay mineral contained in oily gel composition 2-3 had a platy structure with an average thickness of 0.1 µm or less and an average major axis of about 5 µm and with good dispersibility.

The particle shape of the organically-modified clay mineral contained in oily gel composition 2-4 was granular, and the shape was different from that of the above-described organically-modified clay mineral having a platy particle structure.
In the organically-modified clay mineral having the above-described platy particle structure, the platy particle surface (flat side) tends to be hydrophobic, and the end surface tends to be hydrophilic. Therefore, the organically-modified clay mineral having the above-described platy particle structure is arranged, in silicone oil, so that the exposure of the hydrophilic end surface decreases. If schematically shown, it is considered that a gel network shown in Fig. 3 is formed and a high swelling property is achieved.
On the other hand, in the granular particles of the organically-modified clay mineral, it is considered that a gel network by the contribution of hydrophilic and hydrophobic portions is difficult to be formed and that the swelling property is poor.

The platy particles of the organically-modified clay mineral contained in oily gel compositions 2-1 to 2-3 have an different average major axis length and a different degree of dispersibility depending upon treatment time. It is clear from the above-described results that the excellent swelling property can be obtained by the delamination with a treatment time of about 15 minutes or longer.

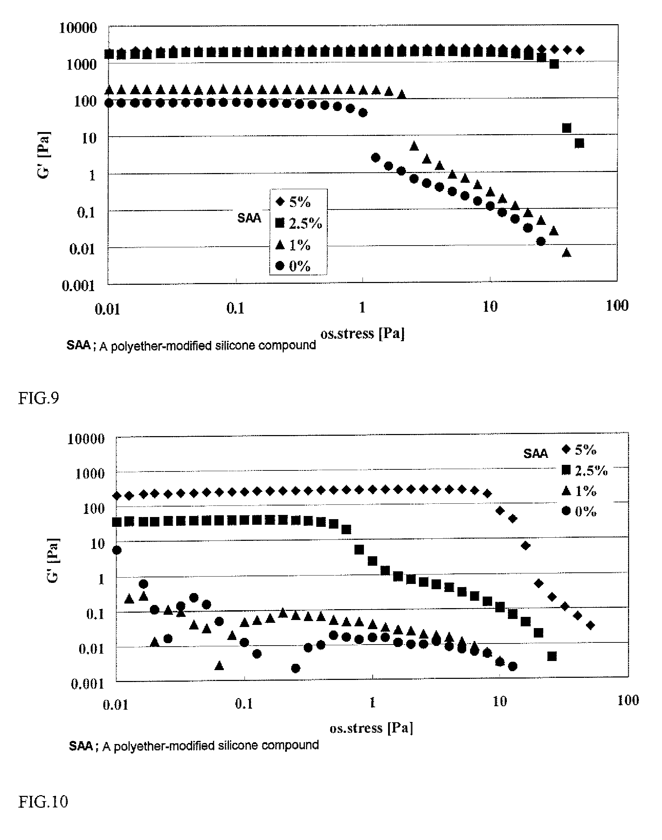

In addition, the stability of gel structure for oily gel compositions was evaluated from dynamic viscoelasticity.
The relationship between the storage modulus (G') and the shear stress for oily gel composition 2-3 (organically-modified clay mineral: platy particles) of the present invention is shown in Fig. 8, and the relationship for oily gel composition 2-4 (organically-modified clay mineral: aggregate) is shown in Fig. 9. In respective figures, the cases in which the concentration of polyether-modified silicone compound contained in respective oily gel compositions was varied are also shown (the increase and decrease were adjusted with decamethylcyclopentasiloxane).

As is for oily gel composition 2-3 of the present invention, when the organically-modified clay mineral particles are platy, it was confirmed that the gel elasticity was high within the fixed shear stress range, a constant state was maintained, and the stable gel structure was taken (Fig. 8).
On the other hand, as is for oily gel composition 2-4, when the granular organieally-modified clay mineral (aggregate) by the normal dispersion treatment of a commercial organically-modified clay mineral is used, the gel elasticity was small, and a stable region with a constant gel elasticity state was scarce compared with the above-described case in Fig 8 (Fig. 9).

From the above results, the organically-modified clay mineral having a platy particle structure of the present invention can be formed by the delamination, in oil, of the organically-modified clay mineral aggregate in the presence of a nonionic surfactant, and it achieves an excellent effect in the formation of a stable gel structure. Thus, it is very clear that the thickening and gelling effect is improved compared with the organically-modified clay mineral with the conventional aggregate structure.

### Oil dispersions 2-1 to 2-8

In addition, the swelling property of organically-modified clay minerals in oil was investigated by changing the amount of added nonionic surfactant. The results are shown in Table 4. In addition, the swelling appearances are shown in Fig. 10. The production methods were as follows.

### (Oil dispersions 2-1 to 2-4)

As the organically-modified clay mineral, a commercial organically-modified hectorite (Bentone 38 VCG, manufactured by Elementis Specialties, Inc. (UK)) was used. The production was carried out, with a Disper disperser, in the same way as production method of the above-described oily gel composition 2-4 (normal dispersion treatment). The organically-modified clay mineral in the obtained oil dispersion was present as granular aggregates.

### (Oil dispersions 2-5 to 2-8)

As the organically-modified clay mineral, a commercial organically-modified hectorite (Bentone 38 VCG, manufactured by Elementis Specialties, Inc. (UK)) was used. The production was carried out, with a bead mill, in the same way as production method of the above-described oily gel composition 2-3 (high-dispersion treatment). The organically-modified clay mineral in the obtained oil dispersion was present as microscopic platy particles.

**[Table 4]**

| | Oil dispersion (normal dispersion treatment) | | | | Oil dispersion (high-dispersion treatment) | | | |
|---|---|---|---|---|---|---|---|---|
| | 2-1 | 2-2 | 2-3 | 2-4 | 2-5 | 2-6 | 2-7 | 2-8 |
| Organically-modifed hectorite *1 (aggregate) | 5.0 | 5.0 | 5.0 | 5.0 | - | - | - | - |
| Organically-modified hectorite *1 (platy particles) | - | - | - | - | 5.0 | 5.0 | 5.0 | 5.0 |
| Polyether-modified silicone compound *2 | 0.0 | 1 0 | 2.5 | 5.0 | 0.0 | 1 .0 | 2.5 | 5.0 |
| Decamethylcyclopentasiloxane | to 100 | to 100 | to 100 | to 100 | to 100 | to 100 | to 100 | to 100 |
| Swelling property | × | × | × | △ | × | △ | ○ | ○ |

As is clear from Table 4, when the aggregated organically-modified hectorite by the normal dispersion treatment was used, the swelling hardly took place without the addition of a polyether-modified silicone compound, which is a nonionic surfactant (dispersion 2-1). When the added amount was increased, a gradual change was observed. However, a reasonable swelling property was observed only after reaching 5 mass % (dispersion 2-4).
On the other hand, when the organically-modified hectorite having a platy particle structure obtained by high-dispersion treatment with a bead mill was used, a certain higher level of swelling property could be achieved, as is clear from Fig. 10, without the addition of a polyether-modified silicone compound than the case in which the aggregate was used. When the amount of added polyether-modified silicone compound exceeded 1 mass %, a satisfactory swelling property was observed.
From the above results, it is also clear that the swelling property is extremely high for the oily gel composition containing the organically-modified clay mineral having a platy structure compared with the oily gel composition containing the aggregate of the organically-modified clay mineral.

### III. W/O emulsion compositions

In the following, a comparison was made, for W/O emulsion compositions, between the organically-modified clay mineral platy particles of the present invention and the conventional organically-modified clay mineral aggregate.

### Emulsions 1-1 to 1-4

W/O emulsions that have compositions shown in Table 5 were prepared. The preparation methods were as follows.

### (Emulsions 1-1 and 1-2)

Commercial organically-modified hectorite and a half quantity of silicone oil were mixed, the dispersion treatment was carried out with a Disper disperser for 15 minutes, and an oily sol composition was obtained. To this, a polyether-modified silicone compound and the rest of the silicone oil were added, the further dispersion treatment was carried out with a Disper disperser for 10 minutes, and an oily gel composition was obtained. The organically-modified clay mineral in this oily gel composition was present as aggregates similar to those in the above-described oily gel composition 1-4.
A W/O type emulsion composition was obtained by emulsification with a Disper disperser while gradually adding ion-exchanged water to the obtained oily gel composition.

### (Emulsions 1-3 and 1-4)

Commercial organically-modified hectorite and a half quantity of silicone oil were mixed, the same volume of glass beads (diameter: 1 mm) as this mixture was added, the dispersion treatment was carried out with a bead mill for 15 minutes, and an oily sol composition was obtained. To this, a polyether-modified silicone compound and the rest of the silicone oil were added, the further dispersion treatment was carried out with a Disper disperser for 10 minutes, and an oily gel composition was obtained. The organically-modified clay mineral in this oily gel composition was present as microscopic platy particles similar to those in the above-described oily gel composition 1-3.
A W/O type emulsion composition was obtained by emulsification with a Disper disperser while gradually adding ion-exchanged water to the obtained oily gel composition.

The evaluation method of the emulsion stability for W/O emulsion compositions and the evaluation criteria are shown below.

### (Stability)

Samples were stored under respective conditions, and the change in appearance was visually evaluated according to the below-described evaluation criteria.
A: No change such as the separation of a liquid phase or aggregation is observed.
B: Some separation is observed in the surface layer.
C: 1/5 or less of the total emulsion composition is separated.
D: 1/5 or more of the total emulsion composition is separated.

**[Table 5]**

| | | Emulsion (normal dispersion treatment) | | | | | | Emulsion (high-dispersion treatment) | | | | | |
|---|---|---|---|---|---|---|---|---|---|---|---|---|---|
| | | 1-1 | | | 1-2 | | | 1-3 | | | 1-4 | | |
| Organically-modified hectorite *1 (aggregate) | | 0.5 | | | 1.0 | | | - | | | - | | |
| Organically-modified hectorite *1 (platy particles) | | - | | | - | | | 0.5 | | | 1 .0 | | |
| Polyether-modifed silicone compound *2 | | 1.0 | | | 1.0 | | | 1.0 | | | 1.0 | | |
| Decamethylcyclopentasiloxane | | to 100 | | | to 100 | | | to 100 | | | to 100 | | |
| Ion exchanged water | | 30.0 | | | 30.0 | | | 30.0 | | | 30.0 | | |
| Stability | Next day (0°C, Room Temperature, 50°C) | A | A | B | A | A | B | A | A | A | A | A | A |
| | 1 week after (0°C. Room Temperature, 50°C) | C | C | D | C | B | C | B | C | C | A | B | B |
| | 1 month after (0°C, Room Temperature, 50°C) | D | D | D | C | C | C | C | C | C | B | B | B |

As is clear from the results in Table 5, the long-term stability of emulsions 1-1 and 1-2, which were prepared using a commercial organically-modified clay mineral in an aggregated state, was poor. In particular, when the content of the organically-modified clay mineral was 0.5 mass % (emulsion 1-1), the viscosity was extremely low, and it was difficult to maintain stable emulsion. When the content was increased to 1 mass % (emulsion 1-2), an increase in viscosity was observed; however, the long-term stability was not satisfactory.
On the other hand, in the use of an organically-modified clay mineral having a platy particle structure, when the blending quantity was 0.5 mass % (emulsion 1-3), the stability was good, in spite of low viscosity, compared with emulsion 1-1 in which the equal blending quantity of the aggregated clay mineral was used. When the blending quantity was increased to 1 mass % (emulsion 1-4), the viscosity became extremely high and the long-term stability became excellent.

From the above results, it was clarified that the viscosity could be improved with a less blending quantity and a W/O type emulsion composition excellent in stability could be obtained by converting aggregated organically-modified clay mineral into a platy structure.

In addition, the emulsification by organically-modified clay mineral itself without blending a polyether-modified silicone compound into a W/O type emulsion composition was investigated.

### Emulsions 1-5 to 1-9

W/O emulsions that have compositions shown in Table 6 were prepared. Emulsions 1-5 and 1-6 were prepared according to the method for the above-described emulsions 1-1 and 1-2 (organically-modified clay mineral: aggregate). Emulsions 1-7 to 1-9 were prepared according to the method for the above-described emulsions 1-3 and 1-4 (organically-modified clay mineral: platy particles). The measurement methods for the viscosity and the emulsion particle size were as follows.

### (Viscosity)

After still standing, for 1 hour, in a constant temperature bath at 30 °C, the measurement was carried out with a single cylindrical rotational viscometer (Shibaura Systems Co., Ltd.) at 12 rpm (rotor No. 3 or No. 4).

### (Emulsion particle size)

The emulsion particle size was measured with an optical microscope (OLYMPUS BM60) for samples that have been stored under respective conditions.

**[Table 6]**

| | Emulsion (normal dispersion treatment) | | Emulsion (high-dispersion treatment) | | |
|---|---|---|---|---|---|
| | 1-5 | 1-6 | 1-7 | 1-8 | 1-9 |
| Orgarically-modified hectorite *1 (aggregate) | 1.0 | 1 .5 | - | - | - |
| Organically-modfied hectorite *1 (platy particles) | - | - | 0.5 | 1.0 | 1.5 |
| Polyether-modified silicone compound *2 | - | - | - | - | - |
| Decamethylcycioxntasiloxsne | to 100 | to 100 | to 100 | to 100 | to 100 |
| Ion exchanged water | 30.0 | 30.0 | 30.0 | 30.0 | 30.0 |
| Emulsion particle size (µ m) of next day | Non-emulsion particles | Non-emulsion particles | 15 | 10 | 10 |
| Viscosity (mPa·s) of next day *rotor No. 3 | up to 100 | up to 100 | 130 | 280 | 630 |
| stability of next day (room temperature) | D | D | D | D | C |

As is clear from the results in Table 6, when the organically-modified clay mineral platy particles of the present invention were used, without blending a polyether-modified silicone compound and only with clay mineral, a certain degree of viscosity adjustment was possible and the emulsification function could be achieved as shown for emulsions 1-7 to 1-9.
On the other hand, when a commercial aggregate organically-modified clay mineral was used in an aggregated state, as shown for emulsions 1-5 and 1-6, reasonable viscosity could not be generated by itself and the emulsion stability was poor.
From the above results, it was confirmed that the organically-modified clay mineral of the present invention having a platy structure could achieve, by itself, thickening and gelling functions without adding other activators. However, it is preferable to blend a polyether-modified silicone compound in order to maintain good stability.

This trend is also similar in the W/O emulsion of the oily gel composition that is obtained, in one step without going through a sol composition, by the delamination of an organically-modified clay mineral, in oil, under the presence of a nonionic surfactant (refer to the below-described emulsions 2-1 to 2-4).

### Emulsions 2-1 to 2-4

W/O emulsions that have compositions shown in Table 7 were prepared. The preparation methods were as follows.

### (Emulsions 2-1 to 2-2)

Commercial organically-modified hectorite, polyether-modified silicone compounds, and silicone oil were mixed, the dispersion treatment was carried out with a Disper disperser for 15 minutes, and an oily gel composition was obtained. The organically-modified clay mineral in this oily gel composition was present as aggregates similar to those in the above-described oily gel composition 2-4.
A W/O type emulsion composition was obtained by emulsification with a Disper disperser while gradually adding ion-exchanged water to the obtained oily gel composition.

### (Emulsions 2-3 and 2-4)

Commercial organically-modified hectorite, polyether-modified silicone compounds, and silicone oil were mixed, the same volume of glass beads (diameter: 1 mm) as this mixture was added, the dispersion treatment was carried out with a bead mill for 15 minutes, and an oily gel composition was obtained. The organically-modified clay mineral in this oily gel composition was present as microscopic platy particles similar to those in the above-described oily gel composition 2-3.
A W/O type emulsion composition was obtained by emulsification with a Disper disperser while gradually adding ion-exchanged water to the obtained oily gel composition.

**[Table 7]**

| | | Emulsion (normal dispersion treatment) | | | | | | Emulsion (high-dispersion treatment) | | | | | |
|---|---|---|---|---|---|---|---|---|---|---|---|---|---|
| | | 2-1 | | | 2-2 | | | 2-3 | | | 2-4 | | |
| Organically-modified hectorite *1 (aggregate) | | 0.5 | | | 1 .0 | | | - | | | - | | |
| Organically-modifed hectorite *1 (platy particles) | | - | | | - | | | 0.5 | | | 1.0 | | |
| Polyether-modified silicone compound *2 | | 1.0 | | | 1.0 | | | 1.0 | | | 1.0 | | |
| Decamethylcyclopentesiloxana | | to 100 | | | to 100 | | | to 100 | | | to 100 | | |
| lon exchanged water | | 30.0 | | | 30.0 | | | 30.0 | | | 30.0 | | |
| Emulsion Particle size (µ m) of 1 month after | | 1-12.5 | | | 1-12.5 | | | 1-12.5 | | | 1-12.5 | | |
| Viscosity | Next day (mPa·s) *rotor No. 3 | up to 100 | | | 860 | | | 120 | | | 1840 | | |
| | 1 week after (mPa·s) | up to 100 | | | 800 | | | 110 | | | 1710 | | |
| | 1 month after (mPa· s) | up to 100 | | | 790 | | | up to 100 | | | 1620 | | |
| Stability | Next day (0°C, Room Temperature. 50°C) | A | A | B | A | A | B | A | A | A | A | A | A |
| | 1 week after (0°C, Room Temperature, 50°C) | C | C | D | C | B | C | B | C | C | A | B | B |
| | 1 month after (0°C, Room Temperature, 50°C) | D | D | D | C | C | C | C | C | C | B | B | B |

As is clear from the results in Table 7, the long-term stability of emulsions 2-1 and 2-2, which were prepared using a commercial organically-modified clay mineral in an aggregated state, was poor. In particular, when the content of the organically-modified clay mineral was 0.5 mass % (emulsion 2-1), the viscosity was extremely low, and it was difficult to maintain stable emulsion. When the content was increased to 1 mass % (emulsion 2-2), an increase in viscosity was observed; however, the long-term stability was not satisfactory.
On the other hand, in the W/O emulsion composition in which an organically-modified clay mineral having a platy particle structure was used, when the blending quantity was 0.5 mass % (emulsion 2-3), the stability was good, in spite of low viscosity, compared with emulsion 2-1 in which the equal blending quantity of the aggregated clay mineral was used. When the blending quantity was increased to 1 mass % (emulsion 2-4), the viscosity became extremely high and the long-term stability became excellent.

From the above results, it was clarified that the viscosity could be improved with a less blending quantity and that a W/O type emulsion composition excellent in stability could be obtained by using an oily gel composition containing the organically-modified clay mineral that was obtained by converting aggregated organically-modified clay mineral into a platy structure.

The investigation was further carried out by varying the type of nonionic surfactant and the type of oil that are blended in the oily gel composition. The results are shown in Tables 8 and 9.

### Emulsions 2-5 to 2-8

The preparation method for W/O emulsions shown in Table 8 was as follows.

### (Emulsions 2-5 to 2-8)

Organically-modified hectorite, nonionic surfactant, and oil were mixed, the same volume of glass beads (diameter: 1 mm) as this mixture was added, the dispersion treatment was carried out with a bead mill for 15 minutes, and an oily gel composition was obtained. The organically-modified clay mineral in this oily gel composition was present as microscopic platy particles similar to those in the above-described oily gel composition 2-3.
A W/O type emulsion composition was obtained by emulsification with a Disper disperser while gradually adding ion-exchanged water to the obtained oily gel composition.

**[Table 8]**

| | Emulsion (high-dispersion treatment) | | | |
|---|---|---|---|---|
| | 2-5 | 2-6 | 2-7 | 2-8 |
| Organically-modified hectorite *1 (platy particles) | 1.0 | 1.0 | 1.0 | 1.0 |
| Polyether-modified silicone compound *2 | 1.0 | - | 1.0 | 1.0 |
| Glyceryl Diisostearate | - | 1.0 | - | - |
| Decamethylcyclopentasiloxane | to 100 | to 100 | - | - |
| Liquid paraffin | - | - | to 100 | - |
| Glyceryl tri-2-ethylhexanoete | - | - | - | to 100 |
| Ion exchanged water | 30.0 | 30.0 | 30.0 | 30.0 |
| Stability of next day (Room temperature) | A | B | C | B |

As is clear from the results in Table 8, in emulsion 2-6, in which the nonionic surfactant was changed, and emulsions 2-7 and 2-8, in which the oil was changed to hydrocarbon oil and ester oil, the stabilities of W/O type emulsion compositions were maintained at a certain level. When the organically-modified clay mineral of the present invention having a platy structure was used, the best stability could be maintained in silicone oil. Accordingly, the application to various types of W/O type emulsion compositions, and in particular, to cosmetics in which the blending of silicone oil is the mainstream is expected.

### Emulsions 1-10 to 1-13

The preparation method for W/O emulsions shown in Table 9 was as follows.

### (Emulsions 1-10 to 1-13)

Commercial organically-modified hectorite and a half quantity of oil were mixed, the same volume of glass beads (diameter: 1 mm) as this mixture was added, the dispersion treatment was carried out with a bead mill for 15 minutes, and an oily sol composition was obtained. To this, a nonionic surfactant and the rest of oil were added, the further dispersion treatment was carried out with a Disper disperser for 10 minutes, and an oily gel composition was obtained. The organically-modified clay mineral in this oily gel composition was present as microscopic platy particles similar to those in the above-described oily gel composition 1-3.
A W/O type emulsion composition was obtained by emulsification with a Disper disperser while gradually adding ion-exchanged water to the obtained oily gel composition.

**[Table 9]**

| | Emulsion (high-dispersion treatment) | | | |
|---|---|---|---|---|
| | 1-10 | 1-11 | 1-12 | 1-13 |
| Organically-modified hectorite *1 (platy particles) | 1.0 | 1.0 | 1.0 | 1.0 |
| Polyether-modified silicone compound *2 | 1.0 | - | 1.0 | 1.0 |
| Glyceryl Diisostearate | - | 1.0 | - | - |
| Decamethylcyclopentasiloxane | to 100 | to 100 | - | - |
| Liquid paraffin | - | - | to 100 | - |
| Glyceryl tri-2-ethylhexanoate | - | - | - | to 100 |
| Ion exchanged water | 30.0 | 30.0 | 30.0 | 30.0 |
| Emulsion particle size (µ m) of next day | 10 | 7.5 | 50 | 12.5 |
| Viscosity (mPa·s) of next day *rotor No. 3 | 280 | 800 | 460 | 3,140 |
| Stability of next day (room temperature) | A | B | C | B |

As is clear from the results in Table 9, in emulsion 1-11, in which the nonionic surfactant was changed, and emulsions 1-12 and 1-13, in which the oil was changed to hydrocarbon oil and ester oil, the stabilities of W/O type emulsion compositions were maintained at a certain level. When the organically-modified clay mineral of the present invention having a platy structure was used, the best stability could be maintained in silicone oil. Accordingly, the application to various types of W/O type emulsion compositions, and in particular, to cosmetics in which the blending of silicone oil is the mainstream is expected.

Hereinafter, examples of cosmetics, in which a W/O type emulsion composition of the oily gel composition containing the organically-modified clay mineral having a platy structure of the present invention was used, are shown. However, the present invention is not limited to these examples.

| Cosmetic 1-1 Sunscreen | |
|---|---|
| (1) Sol composition of dimethyl stearyl ammonium hectorite / Decamethylcyclopentasiloxane (1/20) | 21 |
| (2) Trimethylsiloxysilicate | 1 |
| (3) Polyoxyethylene·methylpolysiloxane copolymer (general formula (I) type, polyoxyethylene content: about 20%, viscosity: 400 to 800 cs, HLB:4.5, R'=H, p=3, y=0) | 0.5 |
| (4) Squalane | 5 |
| (5) Silicone coated micro titanium dioxide (20nm) | 10 |
| (6) Talc (hydrophobized article) | 6 |
| (7) 2-Ethylhexyl-p-methoxycinnamate | 7 |
| (8) Glyceryl ethylhexanoate di-p-methoxycinnamate | 0.5 |
| (9) Spherical polyethylene powder | 5 |
| (10) Dipropylene glycol | 4 |
| (11) Trisodium edetate | 0.02 |
| (12) Paraben | proper quantity |
| (13) Phenoxyethanol | proper quantity |
| (14) Purified water | remainder |
| (15) Perfume | proper quantity |

### (Preparation)

Into the sol dispersion (1) dispersed to microscopic particles with such as a bead mill, (2)-(9) were added , and mixed and stirred. And objective sunscreen were obtained by adding thereto (10)-(14), as water phase parts, to be emulsified, and then adding thereto (15).

| Cosmetic 1-2 Sunscreen | |
|---|---|
| (1) Sol composition of dimethyl stearyl ammonium hectorite / Decamethylcyclopentasiloxane (1/20) | 21 |
| (2) Trimethylsiloxysilicate | 1 |
| (3) Polyoxyethylene·methylpolysiloxane copolymer (general formula (V) type, polyoxyethylene content: about 19%, viscosity: 200 to 700 cs, HLB: 2-4, R'=H, p=3, y=0) | 0.3 |
| (4) Cetyl 2-ethyl hexanoate | 3 |
| (5) Metylpolysiloxane | 5 |
| (6) Silicone coated micro titanium dioxide (20nm) | 5 |
| (7) Silicone coated micro zinc dioxide (20nm) | 10 |
| (8) 2-Ethylhexyl-p-methoxycinnamate | 5 |
| (9) 4-tert-Butyl-4'-methoxydibenzoylmethane | 0.5 |
| (10) Spherical polyethylene powder | 5 |
| (11) 1,3-butylene glycol | 4 |
| (12) Trisodium edetate | 0.02 |
| (13) Paraben | proper quantity |
| (14) Phenoxyethanol | proper quantity |
| (15) Purified water | remainder |
| (16) Perfume | proper quantity |

### (Preparation)

Into the sol dispersion (1) dispersed to microscopic particles with such as a bead mill, (2)-(10) were added, and mixed and stirred. And objective sunscreen were obtained by adding thereto (11)-(15), as water phase parts, to be emulsified, and then adding thereto (16).

| Cosmetic 1-3 Moisture cream | |
|---|---|
| (1) Sol composition of dimethyl stearyl ammonium hectorite / Decamethylcyclopentasiloxane (2/18) | 20 |
| (2) Petrolatum | 2 |
| (3) Microcrystalline wax | 3 |
| (4) Dimethylpolysiloxane (5.6cs/25°C) | 5 |
| (5) Squalane | 3 |
| (6) Polyoxyethylene·methylpolysiloxane copolymer (general formula (I) type, polyoxyethylene content: about 20%, viscosity: 400 to 800 cs, HLB:4.5, R'=H, p=3, y=0) | 1.5 |
| (7) Isostearic acid | 1 |
| (8) Cetyl 2-ethyl hexanoate | 3 |
| (9) Glycerin | 10 |
| (10) 1,3-butylene glycol | 4 |
| (11) Xylit | 2 |
| (12) Sodium chloride | 0.5 |
| (13) Sodium hexametaphosphate | 0.05 |
| (14) Stearyl glycyrrhetinate | 0.05 |
| (15) Sodium DL-pyrrolidonecarboxylate | 1 |
| (16) Curcuma extract | 0.1 |
| (17) Trisodium edetate | 0.1 |
| (18) Paraben | proper quantity |
| (19) Purified water | remainder |
| (20) Perfume | proper quantity |

### (Preparation)

Into the sol dispersion (1) dispersed to microscopic particles with such as a bead mill, (2)-(7) were added , and mixed and stirred. And objective sunscreen were obtained by adding thereto (8)-(19), as water phase parts, to be emulsified, and then adding thereto (20).

| Cosmetic 1-4 Emulsified foundation | |
|---|---|
| (1) Sol composition of dimethyl stearyl ammonium hectorite / Decamethylcyclopentasiloxane (1/20) | 21 |
| (2) Dimethylpolysiloxane (5.6cs/25°C) | 15 |
| (3) Polyoxyethylene·methylpolysiloxane copolymer (general formula (I) type, polyoxyethylene content: about 20%, | |
| viscosity: 400 to 800 cs, HLB:4.5, R'=H, p=3, y=0) | 5 |
| (4) Palmitic acid | 0.5 |
| (5) Cetyl 2-ethyl hexanoate | 5 |
| (6) Silicone coated yellow iron oxide | 2 |
| (7) Silicone coated colcothar | 1 |
| (8) Silicone coated black iron oxide | 0.3 |
| (9) Silicone coated titanium dioxide | 10 |
| (10) Silicone coated talc | 1.5 |
| (11) Silicone coated spindle-shaped titanium dioxide | 3 |
| (12) Spherical nylon powder | 1 |
| (13) Macadamia oil fatty acid cholesterol ester | 0.1 |
| (14) DL-α-Tocopheryl acetate | 0.1 |
| (15) Glycerin | 5 |
| (16) 1,3-butylene glycol | 10 |
| (17) Ester paraoxybenzoate | proper quantity |
| (18) Purified water | remainder |
| (19) Perfume | proper quantity |

### (Preparation)

Into the sol dispersion (1) dispersed to microscopic particles with such as a bead mill, (2)-(14) were added , and mixed and stirred. And objective sunscreen were obtained by adding thereto (15)-(18), as water phase parts, to be emulsified, and then adding thereto (19).

| Cosmetic 1-5 Sunscreen | |
|---|---|
| (1) Sol composition of dimethyl stearyl ammonium hectorite / Decamethylcyclopentasiloxane (1/20) | 21 |
| (2) Trimethylsiloxysilicate | 1 |
| (3) Polyoxyethylene·methylpolysiloxane copolymer | |
| (general formula (VI) type,viscosity: 200 to 800 cs, HLB:3-4) | 0.5 |
| (4) Squalane | 5 |
| (5) Silicone coated micro titanium dioxide (20nm) | 10 |
| (6) Talc (hydrophobized article) | 6 |
| (7) 2-Ethylhexyl-p-methoxycinnamate | 7 |
| (8) Glyceryl ethylhexanoate di-p-methoxycinnamate | 0.5 |
| (9) Spherical polyethylene powder | 5 |
| (10) Dipropylene glycol | 4 |
| (11) Trisodium edetate | 0.02 |
| (12) Paraben | proper quantity |
| (13) Phenoxyethanol | proper quantity |
| (14) Purified water | remainder |
| (15) Perfume | proper quantity |

### (Preparation)

Into the sol dispersion (1) dispersed to microscopic particles with such as a bead mill, (2)-(9) were added , and mixed and stirred. And objective sunscreen were obtained by adding thereto (10)-(14), as water phase parts, to be emulsified, and then adding thereto (15).

| Cosmetic 1-6 Emulsified foundation | |
|---|---|
| (1) Sol composition of dimethyl stearyl ammonium hectorite / Decamethylcyclopentasiloxane (1/20) | 21 |
| (2) Dimethylpolysiloxane (5.6cs/25°C) | 15 |
| (3) Polyoxyethylene·methylpolysiloxane copolymer | |
| (general formula (VII) type, | |
| viscosity: 200 to 700 cs, HLB:3-4, R'=H, p=3, y=0) | 5 |
| (4) Palmitic acid | 0.5 |
| (5) Cetyl 2-ethyl hexanoate | 5 |
| (6) Silicone coated yellow iron oxide | 2 |
| (7) Silicone coated colcothar | 1 |
| (8) Silicone coated black iron oxide | 0.3 |
| (9) Silicone coated titanium dioxide | 10 |
| (10) Silicone coated talc | 1.5 |
| (11) Silicone coated spindle-shaped titanium dioxide | 3 |
| (12) Spherical nylon powder | 1 |
| (13) Macadamia oil fatty acid cholesterol ester | 0.1 |
| (14) DL-α-Tocopheryl acetate | 0.1 |
| (15) Glycerin | 5 |
| (16) 1,3-butylene glycol | 10 |
| (17) Ester paraoxybenzoate | proper quantity |
| (18) Purified water | remainder |
| (19) Perfume | proper quantity |

### (Preparation)

Into the sol dispersion (1) dispersed to microscopic particles with such as a bead mill, (2)-(14) were added , and mixed and stirred. And objective sunscreen were obtained by adding thereto (15)-(18), as water phase parts, to be emulsified, and then adding thereto (19).

| Cosmetic 2-1 Sunscreen | |
|---|---|
| (1) Dimethyl stearyl ammonium hectorite / | |
| Polyoxyethylene·methylpolysiloxane copolymer | |
| (general formula (I) type, polyoxyethylene content: about 20%, | |
| viscosity: 400 to 800 cs, HLB:4.5, R'=H, p=3, y=0) | |
| Decamethylcyclopentasiloxane (0.8/0.4/19.8) | 21 |
| (2) Trimethylsiloxysilicate | 1 |
| (3) Dimethylpolysiloxane (5.6cs/25°C) | 5 |
| (4) 2-Ethylhexyl-p-methoxycinnamate | 5 |
| (5) Silicone coated micro titanium dioxide (20nm) | 15 |
| (6) 2-Ethylhexyl-p-methoxycinnamate | 7 |
| (7) Glyceryl ethylhexanoate di-p-methoxycinnamate | 0.5 |
| (8) Spherical polyethylene powder | 5 |
| (9) Dipropylene glycol | 4 |
| (10) Trisodium edetate | 0.02 |
| (11) Paraben | proper quantity |
| (12) Phenoxyethanol | proper quantity |
| (13) Purified water | remainder |
| (14) Perfume | proper quantity |

### (Preparation)

Into the sol dispersion (1) dispersed to microscopic particles with such as a bead mill, (2)-(8) were added , and mixed and stirred. And objective sunscreen were obtained by adding thereto (9)-(13), as water phase parts, to be emulsified, and then adding thereto (14).

| Cosmetic 2-2 Moisture cream | |
|---|---|
| (1) Dimethyl stearyl ammonium hectorite / | |
| Polyoxyethylene·methylpolysiloxane copolymer | |
| (general formula (VI) type,viscosity: 200 to 800 cs, HLB:3-4) / | |
| Decamethylcyclopentasiloxane (2/1/17) | 20 |
| (2) Petrolatum | 2 |
| (3) Dimethylpolysiloxane (5.6cs/25°C) | 5 |
| (4) Squalane | 1 |
| (5) Isostearic acid | 1 |
| (6) Cetyl 2-ethyl hexanoate | 5 |
| (7) Glycerin | 12 |
| (8) 1,3-butylene glycol | 4 |
| (9) Erythritol | 2 |
| (10) Sodium chloride | 0.5 |
| (11) Sodium hexametaphosphate | 0.05 |
| (12) Magnesium L-ascorbyl-2-phosphate | 2 |
| (13) Thiotaurine | 0.1 |
| (16) Curcuma extract | 0.1 |
| (17) Trisodium edetate | 0.1 |
| (18) Paraben | proper quantity |
| (19) Purified water | remainder |
| (20) Perfume | proper quantity |

### (Preparation)

Into the sol dispersion (1) dispersed to microscopic particles with such as a bead mill, (2)-(6) were added , and mixed and stirred. And objective sunscreen were obtained by adding thereto (7)-(17), as water phase parts, to be emulsified, and then adding thereto (18).

| Cosmetic 2-3 Emulsified foundation | |
|---|---|
| (1) Dimethyl stearyl ammonium hectorite / | |
| Polyoxyethylene·methylpolysiloxane copolymer | |
| (general formula (I) type, polyoxyethylene content: about 20%, | |
| viscosity: 400 to 800 cs, HLB:4.5, R'=H, p=3, y=0) / | |
| Decamethylcyclopentasiloxane (1/0.5/19.5) | 21 |
| (2) Dimethylpolysiloxane (5.6cs/25°C) | 5 |
| (3) Palmitic acid | 0.5 |
| (4) Squalane | 5 |
| (5) Dextrin fatty acid treated titanium dioxide | 15 |
| (6) Dextrin fatty acid treated yellow iron oxide | 3 |
| (7) Dextrin fatty acid treated colcothar | 1.5 |
| (8) Dextrin fatty acid treated black iron oxide | 0.5 |
| (9) Metal soap treated talc | 3 |
| (10) Silicone coated spindle-shaped titanium dioxide | 3 |
| (11) Cross-link type silicone powder (trefil E-506) | 0.1 |
| (12) Colcothar coated titanated mica | 0.5 |
| (13) N-Lauroyl-L-lysine | 2 |
| (14) DL-α-Tocopheryl acetate | 0.1 |
| (15) Glycerin | 3 |
| (16) 1,3-butylene glycol | 5 |
| (17) Ester paraoxybenzoate | proper quantity |
| (18) Purified water | remainder |
| (19) Perfume | proper quantity |

### (Preparation)

Into the sol dispersion (1) dispersed to microscopic particles with such as a bead mill, (2)-(13) were added , and mixed and stirred. And objective sunscreen were obtained by adding thereto (14)-(18), as water phase parts, to be emulsified, and then adding thereto (19).

| Cosmetic 2-4 Emulsified foundation | |
|---|---|
| (1) Dimethyl stearyl ammonium hectorite / | |
| Polyoxyethylene·methylpolysiloxane copolymer | |
| (general formula (VII) type, | |
| viscosity: 200 to 700 cs, HLB:3-4, R'=H, p=3, y=0) / | |
| Decamethylcyclopentasiloxane (1/0.5/19.5) | 21 |
| (2) Dimethylpolysiloxane (5.6cs/25°C) | 5 |
| (3) Squalane | 5 |
| (4) Silicone coated titanium dioxide | 12 |
| (5) Silicone coated yellow iron oxide | 3 |
| (6) Silicone coated colcothar | 1.5 |
| (7) Silicone coated black iron oxide | 0.5 |
| (8) Silicone coated sericite | 5 |
| (9) Silicone coated spindle-shaped titanium dioxide | 3 |
| (10) Cross-link type silicone powder (Trefil E-506) | 0.1 |
| (11) Colcothar coated titanated mica | 0.5 |
| (12) Spherical silica | 3 |
| (13) Dipropylene glycol | 3 |
| (14) 1,3-butylene glycol | 5 |
| (15) Ester paraoxybenzoate | proper quantity |
| (16) Purified water | remainder |
| (19) Alcohol | 5 |
| (18) Perfume | proper quantity |

### (Preparation)

Into the sol dispersion (1) dispersed to microscopic particles with such as a bead mill, (2)-(12) were added , and mixed and stirred. And objective sunscreen were obtained by adding thereto (13)-(16), as water phase parts, to be emulsified and then adding thereto (17)-(18).

| Cosmetic 2-1 Sunscreen | |
|---|---|
| (1) Dimethyl stearyl ammonium hectorite / | |
| Polyoxyethylene-methylpolysiloxane copolymer | |
| (general formula (V) type, polyoxyethylene content: about 19%, | |
| viscosity: 200 to 700 cs, HLB:3-4, R'=H, p=3, y=0) | |
| Decamethylcyclopentasiloxane (0.8/0.4/19.8) | 21 |
| (2) Trimethylsiloxysilicate | 1 |
| (3) Dimethylpolysiloxane (5.6cs/25°C) | 5 |
| (4) 2-Ethylhexyl-p-methoxycinnamate | 5 |
| (5) Silicone coated micro titanium dioxide (20nm) | 15 |
| (6) 2-Ethylhexyl-p-methoxycinnamate | 7 |
| (7) Glyceryl ethylhexanoate di-p-methoxycinnamate | 0.5 |
| (8) Spherical polyethylene powder | 3 |
| (9) Dipropylene glycol | 4 |
| (10) Trisodium edetate | 0.02 |
| (11) Paraben | proper quantity |
| (12) Phenoxyethanol | proper quantity |
| (13) Purified water | remainder |
| (14) Perfume | proper quantity |

### (Preparation)

Into the sol dispersion (1) dispersed to microscopic particles with such as a bead mill, (2)-(8) were added , and mixed and stirred. And objective sunscreen were obtained by adding thereto (9)-(13), as water phase parts, to be emulsified, and then adding thereto (14).

### IV. Use of an oil having COOH/OH groups

As described above, the oil dispersion of organically-modified clay mineral platy particles is normally a low viscosity sol when a nonionic surfactant is not contained. When this sol is used as the oil phase of a W/O emulsion composition, satisfactory emulsion stability cannot be achieved.
On the other hand, if a nonionic surfactant is used in combination with this-type of oil dispersion, a high viscosity gel is obtained. If the high viscosity gel is used as the oil phase of a W/O emulsion composition, very high emulsion stability can be achieved.
However, the W/O emulsion composition becomes highly viscous because the oil phase is highly viscous. If this W/O emulsion composition is used for cosmetics, the spreadability will not be satisfactory.

Thus, organically-modified clay mineral oil dispersions that have a relatively low viscosity but can achieve an excellent effect on emulsion stability when used as the oil phase of a W/O emulsion composition were further investigated.
As a result, it was found that an oil having a COOH group and/or OH group in the molecule has a marked effect as explained below.

### Oil dispersions 3-1 to 3-4

Oil dispersions that have compositions shown in Table 10 were prepared. The preparation methods were as follows.

### (Oil dispersions 3-1 to 3-3)

Commercial organically-modified hectorite (Bentone 38 VCG, manufactured by Elementis Specialties, Inc. (UK)) and the components other than sorbitan sesquioleate were mixed, the same volume of zirconia beads (diameter: 1 mm) as this mixture was added, and the treatment was carried out with a bead mill for 15 minutes. To this, sorbitan sesquioleate was added and homogeneously mixed with a Disper disperser and an oil dispersion was obtained. The organically-modified clay mineral in the oil dispersion was present as microscopic platy particles.

### (Oil dispersion 3-4)

Commercial organically-modified hectorite (Bentone 38 VCG, manufactured by Elementis Specialties, Inc. (UK)) and the components other than sorbitan sesquioleate were mixed, this mixture was treated with a Disper disperser for 15 minutes. To this, sorbitan sesquioleate was added and homogeneously mixed with a Disper disperser and an oil dispersion was obtained. The organically-modified clay mineral in the oil dispersion was present as relatively large aggregates.

**[Table 10]**

| | Oil dispersion (high-dispersion treatment) | | | Oil dispersion (normal dispersion treatment) |
|---|---|---|---|---|
| | 3-1 | 3-2 | 3-3 | 3-4 |
| Decamethylcyclopentasiloxane | 96 | 94 | 93 | 93 |
| Organicaliy-modified hectorite *1 (platy particles) | 4 | 4 | 4 | - |
| Organicaliy-modified hectorite *1 (aggregate) | - | - | - | 4 |
| Polyether-modified silicone compound *2 | 0 | 2 | 2 | 2 |
| Sorbitan sesquiisostearate | - | - | 1 | 1 |
| Total | 100 | 100 | 100 | 100 |
| Viscosity (mPa·s) of next day *rotor No.4 | up to 100 | 5,800 | 700 | 700 |
| Swelling property | × | ○ | ○ | × |

As shown in Table 10, when the preparation was carried out by high-dispersion treatment with a bead mill, oil dispersion 3-1 in which only the organically-modified clay mineral was dispersed was a sol of low viscosity and the swelling property was also low. When a nonionic surfactant such as a polyether-modified silicone compound was used in combination with this, a gel composition with very high viscosity and very high swelling property was obtained as shown for oil dispersion 3-2.
When sorbitan sesquioleate was used in combination, the viscosity significantly decreased, as shown for oil dispersion 3-3, while maintaining the high swelling property of the composition.
On the other hand, although the composition is basically the same as the composition of oil dispersion 3-3, in oil dispersion 3-4, which was prepared by the normal dispersion treatment with a Disper disperser, the swelling property was markedly inferior though the viscosity was low.
In addition, W/O emulsion compositions were prepared and compared.

### Emulsions 3-1 to 3-4

W/O emulsion compositions that have the compositions shown in Table 11 were prepared. The preparation methods were as follows.

### (Emulsions 3-1 to 3-3)

An oil dispersion was prepared using commercial organically-modified hectorite (Bentone 38 VCG, manufactured by Elementis Specialties, Inc. (UK)) and other oil phase components, in the same way as the method for the above-described oil dispersions 3-1 to 3-3, with a paint shaker (organically-modified clay mineral: platy particles). An emulsion was obtained by emulsification with a Disper disperser while gradually adding ion-exchanged water to the oil dispersion.

### (Emulsion 3-4)

An oil dispersion was prepared using commercial organically-modified hectorite (Bentone 38 VCG, manufactured by Elementis Specialties, Inc. (UK)) and other oil phase components, in the same way as the method for the above-described oil dispersion 3-4, with a Disper disperser (organically-modified clay mineral: aggregate). An emulsion was obtained by emulsification with a Disper disperser while gradually adding ion-exchanged water to the oil dispersion.

**[Table 11]**

| | Emulsion 3-1 | Emulsion 3-2 | Emulsion 3-3 | Emulsion 3-4 |
|---|---|---|---|---|
| Decamethylcyclopentasiloxane | 86 | 84 | 83 | 83 |
| Organically-modified hectorite *1 (platy particles) | 4 | 4 | 4 | - |
| Organically-modified hectorite *1 (aggregate) | - | - | - | 4 |
| Polyether-modified silicone compounds *2 | 0 | 2 | 2 | 2 |
| Sorbitan sesquiisostearate | - | - | 1 | 1 |
| Ion exchanged water | 10 | 10 | 10 | 10 |
| Total | 100 | 100 | 100 | 100 |
| Viscosity (mPa·s) of next day *rotor No.4 | 5,800 | 12,800 | 11,300 | 22,800 |
| 1 month after (Room Temperature) | D | A | A | C |

As shown in Table 11, in emulsion 3-3, in which the oil phase is the oil dispersion obtained by high-dispersion treatment and with the use of sorbitan sesquioleate, the viscosity of the emulsion decreased and the spreadability increased while maintaining the equivalent emulsion stability to that of the emulsion in which sorbitan sesquioleate was not used (emulsion 3-2).
Although the composition is the same as that of emulsion 3-3, in emulsion 3-4 in which the oil phase is the oil dispersion obtained by the normal dispersion treatment, the emulsion stability was low regardless of markedly high viscosity, and the feelings in use such as spreadability, freshness, and smoothness were also poor compared with emulsion 3-3.

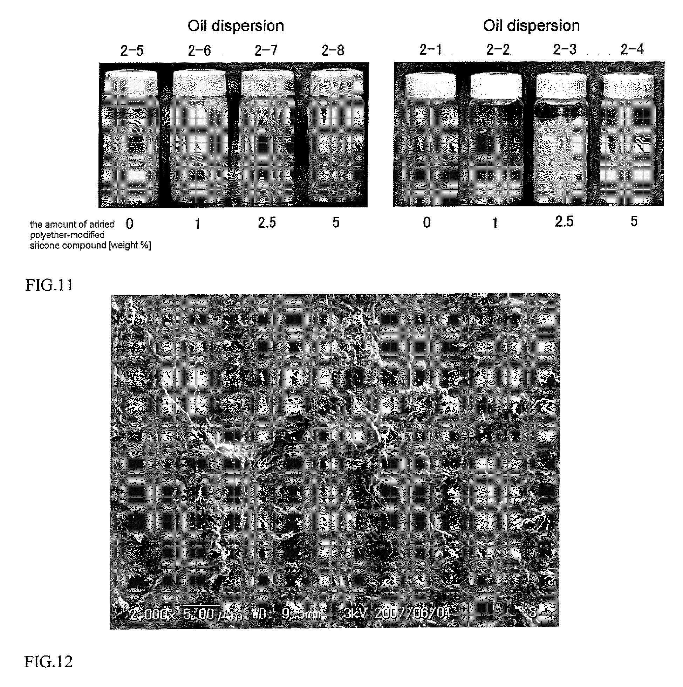

Fig. 11 is an SEM micrograph of oil dispersion 3-4 obtained by the normal dispersion treatment with a Disper disperser. Fig. 12 is an SEM micrograph of oil dispersion 3-3 obtained by the high-dispersion treatment with a bead mill.
In dispersion 3-4 obtained by the normal dispersion treatment, the organically-modified clay mineral was present as relatively large granular aggregates (average particle size: about 20 µm, average thickness: 2µm or higher) as shown in Fig. 11. On the other hand, in dispersions 3-1 to 3-3 obtained by the high-dispersion treatment, the organically-modified clay mineral was present as microscopic platy particles (average thickness: 0.1 µm or less, average major axis: 10 µm or less) as shown in Fig. 12.

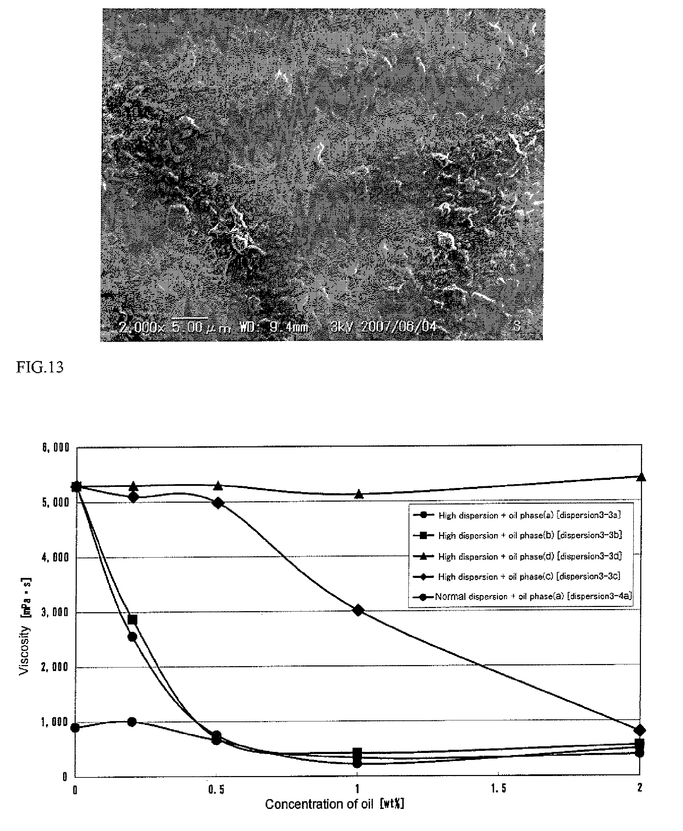

Fig. 13 shows the results for the investigation of the addition effect of various test oils including sorbitan sesquiisostearate.
That is, other oils were used instead of sorbitan sesquioleate in oil dispersion 3-3 (high-dispersion treatment); furthermore, the blending quantity was varied for the preparation of oil dispersions (the increase and decease were adjusted with decamethylcyclopentasiloxane). The viscosities of the obtained oil dispersions were compared (oil dispersions 3-3a to 3-3d). The used test oils a to d were as follows.
Test oil a: sorbitan sesquioleate
Test oil b: 8-methylheptadecanoic acid
Test oil c: isostearyl alcohol
Test oil d: 2-ethylhexyl 2-ethylhexanoate

As seen from Fig. 13, if the oil that has a COOH group or OH group in the molecule, such as test oils a to c, was used in the oil dispersion obtained by high-dispersion treatment, the dispersion viscosity decreased markedly (dispersions 3-3a to 3-3c).
On the other hand, in the case of oil, such as test oil d, that has no COOH group or OH group in the molecule, an increase in the blending quantity hardly changed the viscosity (dispersion 3-3d).

Dispersion 3-4a in Fig. 13 shows the results for the oil dispersions prepared by changing the blending quantity of sorbitan sesquioleate (test oil a) in the above-described oil dispersion 3-4 (normal dispersion treatment). Although the same components are used as those used in dispersion 3-3a, in oil dispersion 3-4a obtained by the normal dispersion treatment, the viscosity lowering effect due to an oil having COOH/OH groups was not observed.

According to the SEM observation, in dispersion 3-4a obtained by the normal dispersion treatment, the organically-modified clay mineral was present as relatively large granules (aggregate), as shown in Fig. 11, at any concentration of the oil having COOH/OH groups. On the other hand, in dispersions 3-3a to 3-3d obtained by the high-dispersion treatment, the organically-modified clay mineral was present as microscopic platy particles, as shown in Fig. 12, at any concentration of the oil having COOH/OH groups.

When an organically-modified clay mineral is delaminated to microscopic platy particles by the high-dispersion treatment, the platy particle surface tends to be hydrophobic, and the platy particle end surface tends to be hydrophilic. Therefore, platy particles are arranged in silicone oil so that the end surface, which is the hydrophilic section, is reduced. As a result, a gel network shown in the schematic figure in Fig. 3 is formed and the swelling property is considered to be exhibited. The particle shape of the organically-modified clay mineral hardly changes depending upon the types of test oils and the concentration. However, the viscosity of dispersion is lowered only by the oil that has a COOH group or OH group in the molecule. Therefore, the viscosity may be considered to be lowered because these oils act on the hydrophilic sites of platy particles of the clay mineral and the interaction is weakened without destroying the gel network among platy particles.

On the other hand, the organically-modified clay mineral is not delaminated and remains as large aggregates in the dispersion obtained by the normal dispersion treatment. Thus, the dispersion state is clearly different from the dispersion obtained by the high-dispersion treatment. Accordingly, hydrophilic sites are hardly exposed in this type of clay mineral aggregate, and the effect of an oil that has a COOH group or OH group in the molecule is hardly present. As a result, it is considered that the lowering of viscosity, which takes place by the high-dispersion treatment, does not take place.

### Emulsions 3-5 to 3-9

In addition, an investigation was carried out by varying the ratio of the water phase to the oil phase for W/O emulsions in which the oil dispersion obtained by the high-dispersion treatment was used as the oil phase. The results are shown in Table 12. The preparation method of the emulsions was as follows.

### (Emulsions 3-5 to 3-9)

A dispersion was obtained by the high-dispersion treatment with a bead mill in the same way as the above-described oil dispersion 3-3. The organically-modified clay mineral in the oil dispersion was present as microscopic platy particles.
This oil dispersion was used as the oil phase. A W/O emulsion composition was obtained by emulsification with a Disper disperser while gradually adding the water phase to the oil phase.

As shown in Table 12, in all W/O emulsion compositions, although the viscosity of the emulsion was low, the emulsion containing an oil having COOH/OH groups emaintained high emulsion stability compared with the emulsion without an oil having COOH/OH groups. In the emulsion spreadability also, the emulsion containing an oil having COOH/OH groups was superior.

**[Table 12]**

| | Emulsion | | | | |
|---|---|---|---|---|---|
| | 3-5 | 3-6 | 3-7 | 3-8 | 3-9 |
| Decamethylcyclopentasiloxane | Remainder | Remainder | Remainder | Remainder | Remainder |
| Organically-modified hectorite *1 (platy particles) | 4 | 4 | 4 | 4 | 4 |
| Polyether-modified silicone compound *2 | 2 | 2 | 2 | 2 | 2 |
| Sorbitan sesquiisostearate (a) | 0 or 1 | 0 or 1 | 0 or 1 | 0 or 1 | 0 or 1 |
| lon exchanged water | 0 | 0.2 | 1 | 10 0 | 20 |
| Total | 100 | 100 | 100 | 100 | 100 |
| Viscosity (mPa·s) *rotor No. 4 | | | | | |
| no containing (a) | 5,800 | 7,000 | 8,200 | 12,800 | 18,700 |
| containing (a) | 700 | 1,500 | 3,600 | 11,300 | 17,000 |
| Stability (One month after, Room temperature) | | | | | |
| no containing (a) | - | A | A | A | A |
| containing (a) | - | A | A | A | A |

### Emulsions 3-10 to 3-12

In addition, an investigation was carried out by varying the ratio of the water phase to the oil phase for W/O emulsions in which the oil dispersion obtained by the normal dispersion treatment was used as the oil phase.
The emulsions in Table 13 are W/O emulsion compositions obtained by using, as the oil phase, the oil dispersion obtained, in the same way as the above-described oil dispersion 3-4, by the normal dispersion treatment (organically-modified clay mineral: aggregate) and by the emulsification with a Disper disperser while gradually adding the water phase to the oil dispersion.
As seen from Table 13, in W/O emulsion compositions in which the oil dispersion obtained by the normal dispersion treatment was used as the oil phase, the viscosity was higher when an oil having COOH/OH groups was blended. This trend was opposite to that in Table 12. The reason for this is not clear; however, it is considered that a difference in the shapes of dispersed organically-modified clay mineral particles is one of the causes.
When a comparison is made among the systems, in which an oil having COOH/OH groups was blended, in Table 12 and Table 13, the spreadability was good at low viscosity, and the feelings in use such as freshness and smoothness were also excellent in the case in which the organically-modifed clay mineral was present as platy particles than the case in which organically-modified clay mineral was present as aggregates.

**[Table 13]**

| | Emulsion | | |
|---|---|---|---|
| | 3-10 | 3-11 | 3-12 |
| Decamathylcyclopentasiloxane | Remainder | Remainder | Remainder |
| Organically-modifed hectorite *1 (aggregate) | 4 | 4 | 4 |
| Polyether-modified silicone compound *2 | 2 | 2 | 2 |
| Sorbitan sesquilsostearate (a) | 0 or 1 | 0 or 1 | 0 or 1 |
| lon exchanged water | 0 | 0.2 | 1 |
| Total | 100 | 100 | 100 |
| Viscosity (mPa·s) *rotor No. 4 | | | |
| no containing (a) | 2,100 | 9,900 | 16,500 |
| containing (a) | 700 | 22,800 | 25,000 |

### Oil dispersions 3-13 and 3-14 and emulsions 3-13 and 3-14

The addition method of an oil having COOH/OH groups was also investigated.
In Table 14, dispersion 3-14 is an oil dispersion of the organieally-modified clay mineral platy particles obtained, in the same way as the above-described dispersion 3-3, by the addition of sorbitan sesquioleate after the high-dispersion treatment of the components other than sorbitan sesquioleate (post-addition).).
On the other hand, dispersion 3-13 is an oil dispersion of the organically-modified clay mineral platy particles obtained by the high-dispersion treatment after mixing sorbitan sesquioleate with other components though the composition is basically the same as that of dispersion 3-14 (pre-addition).

As shown in Table 14, when the oil having COOH/OH groups is added after the high-dispersion treatment of organically-modified clay mineral (post-addition), the viscosity of the oil dispersion was lower compared with the addition before the high-dispersion treatment (pre-addition).

**[Table 14]**

| | Dispersion 3-13 | Dispersion 3-14 |
|---|---|---|
| Decamethylcyclopentasiloxane | 93 | 93 |
| Organically-modified hectorite *1 (aggregate) | 4 | 4 |
| Polyether-modified silicone compound *2 | 2 | 2 |
| Sorbitan sesquiisostearate (a) | 1 | 1 |
| Total | 100 | 100 |
| Adding method | pre-addition | post-addition |
| Viscosity (mPa·s) *rotor No.3 | 1,010 | 360 |

Table 15 lists W/O emulsion compositions obtained by emulsification with a Disper disperser while gradually adding 25 mass portion of ion-exchanged water to 75 mass portion of dispersion 3-13 or dispersion 3-14.
As shown in Table 15, the viscosity markedly decreased and the average size of emulsion particles became smaller in emulsion 3-14 wherein dispersion 3-14, which was obtained by the post-addition of an oil with COOH/OH groups, was used than emulsion 3-13 wherein dispersion 3-13, which was obtained by the pre-addition of an oil with COOH/OH groups, was used.
Accordingly, in order to satisfactorily achieve the effect of an liquid oil with a COOH group and/or OH group in the present invention, it is preferable to add the oil after the high-dispersion treatment of the organically-modified clay mineral.

**[Table 15]**

| | Emulsion 3-13 | Emulsion 3-14 |
|---|---|---|
| Oil dispersion 3-13 (pre-addition (a)) | 75 | - |
| Oil dispersion 3-14 (post-addition (a)) | - | 75 |
| Ion exchanged water | 25 | 25 |
| Emulsions particle size (average value) (µm) | 1-10 (20) | 1-7.5 (15) |
| Viscosity (mPa·s) | 42500 * | 33500 * |

| | | |
|---|---|---|
| * carried out with rotor No.3 | | |

### Cosmetics 3-1 and 3-2, W/O foundation

W/O foundations that have compositions shown in Table 16 were produced. The production method was as follows.

### (Cosmetic 3-1)

Into the sol dispersion obtained by the high-dispersion treatment, with a bead mill using glass beads, of an organically-modified clay mineral in silicone oil, other oil components and powder were added, respectively, and the dispersion was carried out with a homomixer. This oil dispersion was used as the oil phase, and a W/O foundation was obtained by emulsification with a homomixer while gradually adding the water phase to the oil phase

### (Cosmetic 3-2)

Oil components and powder were added, respectively, and the dispersion was carried out with a homomixer. This oil dispersion was used as the oil phase, and a W/O foundation was obtained by emulsification with a homomixer while gradually adding the water phase to the oil phase.

The feeling in use was evaluated as follows.

### (Feeling in use)

Questionnaires were administered to 10 professional panelists concerning each item of the feeling in use when applied on the skin, and the results were evaluated based on the below-described criteria.
○: 8 to 10 panelists answered that it was good.
△: 5 to 7 panelists answered that it was good.
×: 0 to 4 panelists answered that it was good.

As seen from Table 16, although the compositions are basically the same, in the W/O emulsion cosmetic (cosmetic 3-1) in which a dispersion of organically-modified clay mineral platy particles obtained by the high-dispersion treatment was used, the emulsion stability was high, while the viscosity was low, compared with the W/O emulsion cosmetic (cosmetic 3-2) in which a dispersion of organically-modified clay mineral aggregate obtained by the normal dispersion treatment was used. In addition, the spreadability was light and the freshness and smoothness were excellent. The covering ability and the uniformity in finishing were also excellent.

**[Table 16]**

| No. | Component | Cosmetic 3-1 | Cosmetic 3-2 |
|---|---|---|---|
| 1 | Decamethylcyclopentasiloxane | 32 | 32 |
| 2 | Organically-modified hectorite *1 (platy particles) | 1 | - |
| 2 | Organically-modified hectorite *1 (aggregate) | - | 1 |
| 3 | Polyether-modified silicone compound *2 | 2 | 2 |
| 4 | Sorbitan sesquiisostearate (a) | 1 | 1 |
| 5 | Octyl methoxycinnamate | 2 | 2 |
| 6 | Cross-link type methyl polysiloxane | 8 | 8 |
| 7 | Titanium dioxide | 9.7 | 9.7 |
| 8 | Mellow iron oxide | 2 | 2 |
| 9 | Red iron oxide | 0.6 | 0.6 |
| 10 | Black iron oxide | 0.1 | 0.1 |
| 11 | Ion exchanged water | 31 | 31 |
| 12 | Moisturizer | 10 | 10 |
| 13 | Chelating agent | 1 | 1 |
| 14 | Antisepitic | Proper quantity | Proper quantity |
| Viscosity (mPa·s) current day *rotor No.3 | | 5,800 | 7,100 |
| 1 month after (Room temperature | | 5,800 | 6,500 |
| Stability (One month after, Room temperature) | | A | C |
| Smoothness | | ○ | △ |
| Spreadability | | ○ | △ |
| Freshness | | ○ | △ |
| Covering ability | | ○ | △ |
| Uniformity in finishing | | ○ | △ |

## Claims

1. A sol composition comprising an organically-modified clay mineral, having a platy particle structure with an average thickness of 0.1 µm or less and an average major axis of 0.5 to 50 µm in oil

2. The sol composition according to claim 1, wherein the said organically-modified clay mineral is an organically-modified hectorite.

3. The sol composition according to claim 1 or 2, wherein the oil contains silicone oil.

4. The sol composition according to claim 1 to 3, wherein the production is carried out by delaminating, in oil, the aggregate of organically-modified clay mineral having a layer structure with an average thickness of 2 µm or more with a mechanical shearing force and/or impact force.

5. An oily gel composition comprising the sol composition according to claim 1 to 4 and a nonionic surfactant.

6. An oily gel composition comprising the below-described components (i) to (iii).
(i) Organically-modified clay mineral, having a platy particle structure, with an average thickness of 0.1 µm or less and an average major axis of 0.5 to 50 µm
(ii) Nonionic surfactant
(iii) Oil

7. The oily gel composition according to claim 6, wherein said organically-modified clay mineral having a platy particle structure is an organically-modified hectorite.

8. The oily sol composition according to claim 6 or 7, wherein the oil contains silicone oil.

9. The oily gel composition according to claim 6 to 8, wherein the production is carried out by delaminating, in oil, the aggregate of organically-modified clay mineral having a layer structure with an average thickness of 2 µm or more with a mechanical shearing force and/or impact force in the presence of a nonionic surfactant.

10. The oily gel composition according to claim 6 to 8, wherein the production is carried out by delaminating, in oil, the aggregate of organically-modified clay mineral having a layer structure with an average thickness of 2 µm or more, in the absence of a nonionic surfactant, with a mechanical shearing force and/or impact force, and the subsequent addition of a nonionic surfactant.

11. The oily gel composition according to claim 5 to 10, wherein the nonionic surfactant is a polyether-modified silicone compound.

12. The oily gel composition according to claim 5 to 10, wherein the oil having a COOH group and/or OH group in the molecule is additionally contained.

13. The oily gel composition according to claim 12, wherein the the production is carried out by delaminating, in oil, the aggregate of organically-modified clay mineral having a layer structure with an average thickness of 2 µm or more, in the absence of an oil having a COOH group and/or OH group in the molecule, with a mechanical shearing force and/or impact force, and the subsequent addition of an oil having a COOH group and/or OH group in the molecule.

14. The oily gel composition according to claim 12, wherein the production is carried out by delaminating, in the oil containing an oil having a COOH group and/or OH group in the molecule, the aggregate of organically-modified clay mineral having a layer structure with an average thickness of 2 µm or more with a mechanical shearing force and/or impact force.

15. The oily gel composition according to claim 5 to 14, wherein the blending quantity of said organically-modified clay mineral having a platy particle structure is 0.25 to 30 mass % of the oily gel composition.

16. The oily gel composition according to claim 5 to 14, wherein the blending quantity of the said organically-modified clay mineral having a platy particle structure is 0.25 to 10 mass % of the oily gel composition.

17. The oily gel composition according to claim 5 to 16, wherein the the blending quantity of the said nonionic surfactant is 0.1 to 10 mass % of the oily gel composition.

18. The oily gel composition according to claim 12 to 16, wherein the content of the oil having a COOH group and/or OH group in the molecule is 0.1 to 5 mass % of the oily gel composition.

19. A W/O type emulsion composition comprising the oily gel composition according to any of Claims 5 to 18.

20. A cosmetics comprising the oily gel composition according to any of Claims 5 to 18.
